# EUROPEAN PATENT SPECIFICATION

(11) **EP 1 211 310 B1**
(45) Date of publication and mention of the grant of the patent: **13.12.2006**
(21) Application number: 00953436.3
(22) Date of filing: 16.08.2000
(51) Int. Cl.: C12N 1/19, C12P 21/02, C12N 9/24, C12P 19/18, C12P 21/00

(54) **NOVEL YEAST VARIANTS AND PROCESS FOR PRODUCING GLYCOPROTEIN CONTAINING MAMMALIAN TYPE SUGAR CHAIN**
HEFEVARIANTEN UND VERFAHREN ZUR HERSTELLUNG VON GLYKOPROTEIN ENTHALTENDEN ZUCKERKETTEN VOM SÄUGETIERTYP
NOUVELLES VARIANTES DE LEVURE ET PROCEDE DE PRODUCTION DE GLYCOPROTEINE

(30) Priority: 19.08.1999 JP 23321599
(43) Date of publication of application: 05.06.2002
(73) Proprietor: KIRIN BEER KABUSHIKI KAISHA, Tokyo 104-8288 (JP); National Institute of Advanced Industrial Science and Technology, Tokyo 100-8921 (JP)
(72) Inventor: Chiba, Yasunori, Kiban Gijutsu Kenkyusho, Yokohama-shi, Kanagawa 236-0004 (JP); Kainuma, Mami, Kiban Gijutsu Kenkyusho, Yokohama-shi, Kanagawa 236-0004 (JP); Takeuchi, Makoto, Kiban Gijutsu Kenkyusho, Yokohama-shi, Kanagawa 236-0004 (JP); Kawashima, Nagako Kiban Gijutsu Kenkyusho, Yokohama-shi, Kanagawa 236-0004 (JP); Yoshida, Satoshi, Kiban Gijutsu Kenkyusho, Yokohama-shi, Kanagawa 236-0004 (JP); Yamano, Shigeyuki, Kiban Gijutsu Kenkyusho, Yokohama-shi, Kanagawa 236-0004 (JP); Jigami, Yoshifumi, Ushiku-shi, Ibaraki 300-1234 (JP); Ishii, Tomoko, Tsukuba-shi, Ibaraki 305-0042 (JP); Shimma, Yoh-ichi, Tsukuba-shi, Ibaraki 305-0031 (JP)
(74) Representative: HOFFMANN EITLE
(86) International application number: PCT/JP2000/005474
(87) International publication number: WO 2001/014522

(56) References cited:
- MISAGO M ET AL: "Molecular cloning and expression of cDNAs encoding human alpha-mannosidase II and a previously unrecognized alpha-mannosidase IIx isozyme" PROC. NATL. ACAD. SCI. USA, vol. 92, - December 1995 (1995-12) pages 11766-11770, XP002239755
- NAKAYAMA K. ET AL.: 'OCH1 encodes a novel membrane bound mannosyltransferase: outer chain elongation of asparagine-linked oligosaccharides' EMBO J. vol. 11, 1992, pages 2511 - 2519, XP002934332
- ODANI T. ET AL.: 'Mannosylphosphate transfer to cell wall mannan is regulated by the transcriptional level of the MNN4 gene in saccharomyces cerevisiae' FEBS LETTERS vol. 420, 1997, pages 186 - 190, XP002934333
- NAKANISHI Y. ET AL.: 'Structure of the N-linked oligosaccharides that show the complete loss of alpha-1,6-polymannose outer chain from Och1, Och1 Mnn1 and Och1 Mnn1 Alg3 mutants of saccharomyces cerevisiae' J. BIOL. CHEM. vol. 268, 1993, pages 26338 - 26345, XP002934334
- CHIBA ET AL: 'Production of Human Compatible HIgh Mannose-type (Man5GlcNAc2) SUgar Chains in Saccharomyces cerevisiae' J. BIOL. CHEM. vol. 273, no. 41, 09 October 1998, pages 26298 - 26304
- YOSHIDA: 'Expression and characterization of rat UDP-N-acetylglucosamine: alpha-3-D-mannosidasebta-1, 2-N-acetylglucosaminyltransferase I in Saccharomyces cerevisiae' GLYCOBIOLOGY vol. 9, no. 1, January 1999, pages 53 - 58
- VEROSTEK M.F.; ATKINSON P.H.; TRIMBLE R.B.: 'Glycoprotein biosynthesis in the alg3 Saccharomyces cerevisiae mutant. II. Structure of novel Man6-10GlcNAc2 processing intermediates on secreted invertase.' J. BIOL. CHEM. vol. 268, no. 16, 05 June 1996, pages 12104 - 12115

## Description

### FIELD OF THE INVENTION

The present invention relates to novel yeast mutants capable of producing a glycoprotein in which a sugar chain, having a sugar chain structure identical to that of a sugar chain produced from mammalian cells, is attached to an asparagine residue of protein and a process for producing the sugar chain and the glycoprotein by glycoengineering using the mutants.

### BACKGROUND OF THE INVENTION

There are two types of proteins existing in the natural world, that is, a simple protein consisting of amino acids and a conjugated protein in which, a sugar chain, a lipid, or a phosphoric acid and the like are combined. It is known that most cytokines are glycoproteins. It has been revealed that among these, erythropoietin (EPO), tissue plasminogen activator (TPA) and the like do not exhibit the inherent physiological activities upon removal of the sugar chain (Akira Kobata, TanpakushitsuKakusanKoso, 36, 775-788 (1991)). Thus, sugar chains are expected to play an important role in expressing some biological activities; however, since the correlation between a sugar chain structure and a biological activity is not always clear, development of a technique with which a sugar chain structure (type of sugar, binding site, chain length and the like) that is attached to a protein portion can be freely varied and regulated is necessary.

Sugar chains of glycoprotein are roughly classified into, Asn-linked type, mucin-type, O-GlcNAc type, GPI anchor type, and proteoglycan type and the like (Makoto Takeuchi, Glycobiology Series 5, Glycotechnology, edited by Akira Kobata, Sen-ichiro Hakomori, and Katsutaka Nagai, Kodansha Scientific, 191-208 (1994)), each of which has an inherent pathway for biosynthesis and is in charge of a separate physiological function. Much is known regarding the biosynthetic pathway of an Asn-linked sugar chain and that is examined in detail.

Biosynthesis of an Asn-linked sugar chain is carried out as follows. A precursor comprising N-acetylglucosamine, mannose, and glucose is synthesized on a lipid carrier intermediate and is first transferred to a specific sequence (Asn-X-Ser or Thr) in the glycoprotein on an endoplasmic reticulum (ER). The precursor is then processed (cleavage of a glucose residue and a specific mannose residue), and M8 high mannose type sugar chain (Man₈GlcNAc₂) consisting of 8 mannose residues and 2 N-acetylglucosamine residues is synthesized. Protein containing this high mannose type sugar chain is transported to Golgi apparatus and subjected to various modifications. Modification at the Golgi apparatus for yeast is greatly different from that for mammals (Gemmill, T.R., Trimble, R.B., Biochim. Biophys. Acta., 1426, 227 (1999)).

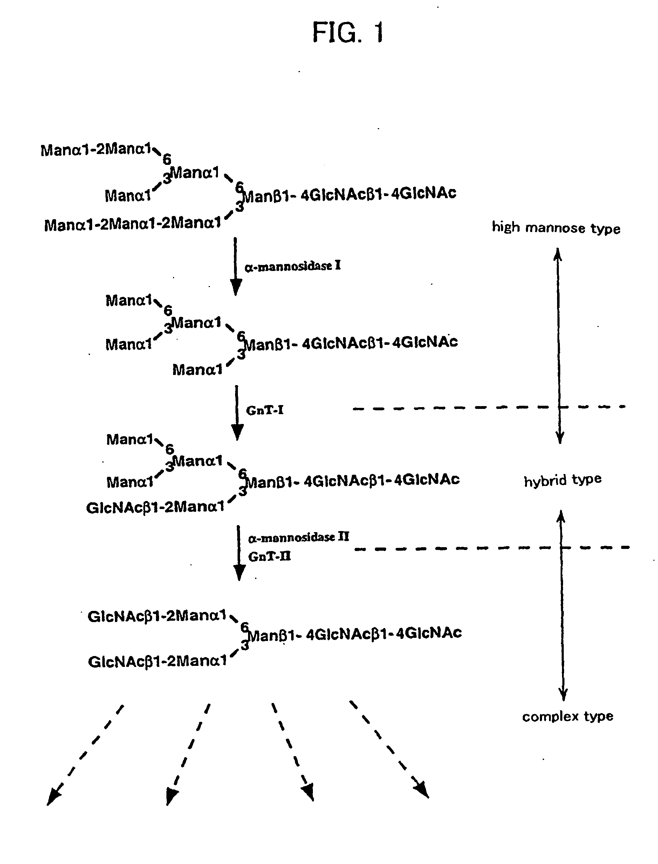

In mammalian cells, α-mannosidase I often acts on the high mannose type sugar chain and cleaves several mannose residues. A sugar chain that is produced in this process (Man₅₋₈GlcNAc₂) is referred to as a high mannose type sugar chain. N-acetylglucosaminyl transferase (GnT) I acts on M5 high mannose type sugar chain (Man₅GlcNAc₂) resulting from the cleavage of 3 mannose residues, whereby one N-acetylglucosamine residue is transferred and a sugar chain consisting of GlcNAcMan₅GlcNAc₂ is produced. The thus obtained sugar chain is referred to as a hybrid type. Further, when α-mannosidase II and GnT-II act, a sugar chain structure that is referred to as a complex type, i.e., GlcNAc₂Man₃GlcNAC₂ is produced. Thereafter, ten-odd types of glycosyltransferases act and attach N-acetylglucosamine, galactose, a sialic acid and the like to form various mammalian type sugar chains (Fig. 1). In mammals, a sugar chain of any of a high-mannose type, hybrid type, or complex type is seen. Sugar chains to be linked differ depending on the type of protein, and, in some cases, different sugar chains are linked within one protein. These sugar chains exhibit excellent functions in biosynthesis of glycoproteins, sorting within a cell, concealing antigenicity, in vivo stability, and organ-targeting properties and the like, depending on the type of sugar chain and the variety oflinked sugar chains (Tamao Endo, Tosa Kogaku (Glycoengineering), Sangyo Chosakai, 64-72 (1992)).

Regarding erythropoietin, which is the first glycoprotein pharmaceutical produced utilizing a recombinant animal cell as a host cell, the importance of the sugar chain is pointed out. The sugar chain of erythropoietin inhibits binding to a receptor; however, the sugar chain definitely contributes to retention of the active structure and improvement of pharmacokinetics. This indicates that the sugar chain of erythropoietin as a whole is essential to expression of pharmacological activity (Takeuchi and Kobata, Glycobiology, 1, 337-346 (1991)). Further, a strong correlation was found between a sugar chain structure, type, number of branches (the number of branches formed by GlcNAc bound to Man₃GlcNAc₂), and a pharmacological effect of erythropoietin (Takeuchi et al., Proc. Natl. Acad. Sci. USA, 86, 7819-7822 (1989)). The main reason for this phenomenon is reported as follows: with an erythropoietin, a branch structure of which is not developed, clearance speed in a kidney is increased and, as a result, retention time in the body becomes shorter (Misaizu et al., Blood, 86, 4097-4104 (1995)). Examples similar to this can be found in a serum glycoprotein such as fetuin and the like, in which the removal of a sialic acid at a sugar chain end exposes galactose, leading to the recognition by a lectin on the surface of a liver cell and rapid disappearance of the glycoprotein from blood (Ashwell and Harford, Annu. Rev. Biochem., 51, 531-554 (1982); Morell et al., J. Biol. Chem., 243, 155-159 (1968)).

Many of the enzyme groups localized in human lysosome are biosynthesized and transported to Golgi apparatus, where a phosphate group is attached to a non-reducing terminal mannose residue at position 6 of the high mannose type sugar chain. This becomes a recognition marker that is specific to a lysosome enzyme. Then through linkage to mannose-6-phosphate receptor (MPR) as the high affinity receptor, the lysosome enzyme is separated from other proteins and transported to a prelysosome. After being dissociated from MPR under acidic conditions, the enzyme is further transported to a lysosome (von Figura and Hasilik, Annu. Rev. Biochem., 54, 167-193 (1984)). The addition reaction of a phosphate group specific to lysosome enzymes is carried out by two types of enzyme reactions. In the case where genes for these reactions have genetic deficiency, it is known that a disorder occurs in a targeting mechanism to lysosome and a serious disease generally referred to as lysosomal disease occurs (Leroy and DeMars, Science, 157, 804-806 (1967)). Therefore, it can be said that the functions of mammalian sugar chains significantly vary depending on their structures.

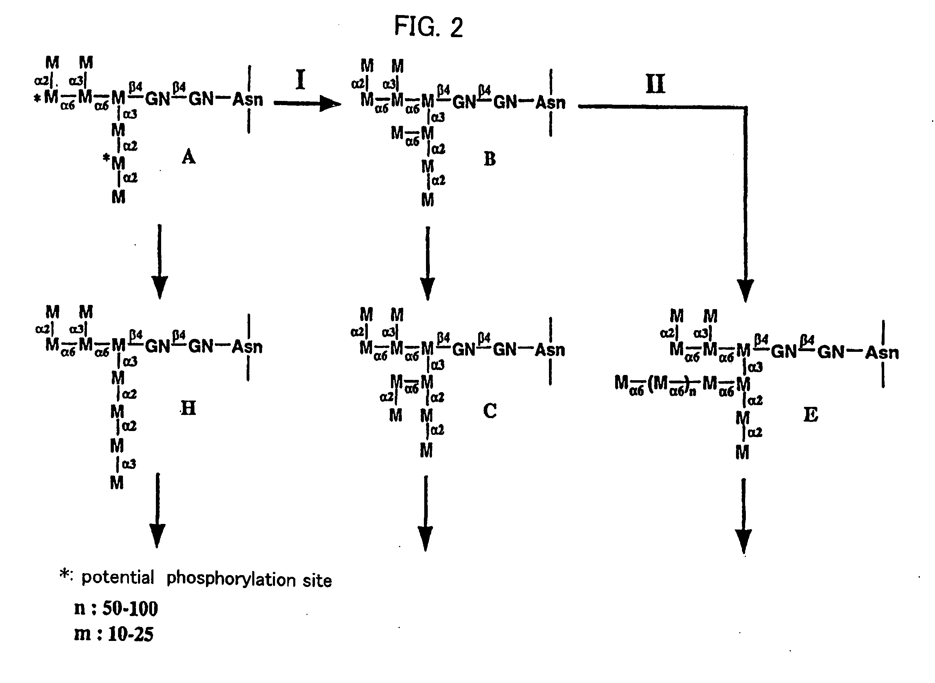

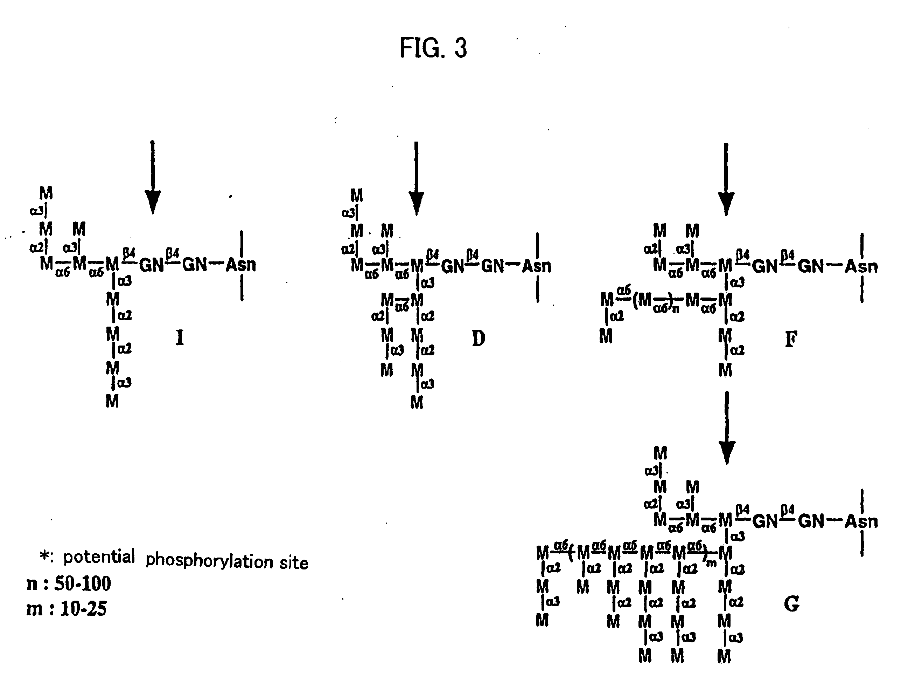

On the other hand, regarding yeast, a mannan type sugar chain (outer sugar chain) is produced in which several to 100 or more mannose residues are attached to M8 high mannose type sugar chain. Biosynthesis of an outer sugar chain in yeast *Saccharomyces* is considered to be performed along a pathway as shown in Figs. 2 and 3 (Ballou et al., Proc. Natl. Acad. Sci. USA, 87, 3368-3372 (1990)). That is, a reaction for initiating elongation begins in which a mannose is first attached to M8 high mannose type sugar chain through α-1,6 linkage (Fig. 2, reaction I, B). It has been clarified that the enzyme performing this reaction is a protein coded by OCH1 gene (Nakayama et al., EMBO J., 11, 2511-2519 (1992)). Further, a stepwise elongation reaction with mannose (Fig. 2, II) through α-1,6 linkage forms poly α-1,6-mannose linkage as a skeleton for an outer sugar chain (Fig. 2, E). To the α-1,6 linked mannose, branched α-1,2 linked mannose may attach (Fig. 2, 3: C, F, H). Additionally, at the end of the branched α-1,2 linked mannose chain, α-1,3 linked mannose may attached (Fig. 2, 3: D, G, H, I). Addition of α-1,3 linked mannose is caused by a MNN1 gene product (Nakanishi-Shindo et al., J. Biol. Chem., 268, 26338-26345 (1993)). Acidic sugar chains can be produced in which a mannose-1-phosphate is attached to a high mannose type sugar chain portion and an outer sugar chain portion (Fig. 2, *; a site capable of being phosphorylated (a potential phosphorylation site) corresponding to * in the above formula (I)). This reaction was found to be caused by a protein that is coded by MNN6 gene (Wang et al., J. Biol. Chem., 272, 18117-18124 (1997)). Further, a gene (MNN4) coding for a protein positively regulating the rearrangement reaction was revealed (Odani et al., Glycobiology, 6, 805-810 (1996) ; Odani et al., FEBS letters, 420, 186-190 (1997)).

In many cases, outer sugar chains produces heterogeneous protein products, and this makes protein purification difficult or lowers the specific activity of proteins (Bekkers et al., Biochim. Biophys. Acta, 1089, 345-351 (1991)). Moreover, since sugar chain structures greatly vary, biological activity identical to that derived from mammals may not be detected in a glycoprotein produced by yeast, or the glycoprotein may have strong immunogenicity against mammals and the like. Hence, as a host in producing useful glycoproteins derived from mammals, yeast is supposedly unsuitable. Accordingly, development of a yeast capable of producing a glycoprotein having biological activity equivalent to that derived from mammals, that is, a glycoprotein containing a mammalian type sugar chain, is desired in academia and industry.

Therefore, in order to produce a mammalian type sugar chain using yeast, it is important to isolate a mutant having a sugar chain biosynthesis system attaching many mannoses that are a modification of a glycoprotein sugar chain specific to the yeast in which, at the outset, the above-described reaction doesn't occur, an outer sugar chain is not attached, and synthesis of sugar chain stops at M8 high mannose type sugar chain. Subsequently, the mammalian type sugar chain could be produced through introduction of biosynthetic genes for sugar chain adding a mammalian type sugar chain to an M8 high mannose type sugar chain, that is a precursor for this mammalian type sugar chain, into the yeast mutant.

In order to obtain a glycoprotein lacking the outer sugar chain, use of a deficient mutant strain for enzymes for producing outer sugar chains in yeast has heretofore been studied. A deficient mutant strain may be provided by obtaining a mutant using drugs, ultraviolet irradiation, or natural mutation, or by artificially disrupting a target gene.

There have been various reports on the former method. For example, mnn2 mutant is defective in the step of branching which causes α-1,2 linkage from an α-1,6 skeleton of an outer sugar chain, and mnn1 mutant is defective in the step of producing α-1,3 linkage mannose at the end of a branch. However, these mutants do not have defects in α-1,6 mannose linkage as a skeleton of an outer sugar chain, and thus, they produce a long outer sugar chain. For example, mnn7, 8, 9, 10 mutants are isolated as mutants having only about 4 to 15 molecules of α-1, 6 linkage mannose. This merely shortens the outer sugar chain of these mutants, but sugar chain elongation does not stop at the high mannose type sugar chain (Ballou et al., J. Biol. Chem., 255, 5986-5991 (1980); Ballou et al., J. Biol. Chem., 264, 11857-11864 (1989)). Defects in addition mechanism of outer sugar chain can be observed in, for example, secretion mutants such as sec18 in which protein transportation from endoplasmic reticulum to Golgi apparatus is temperature-sensitive. However, in a sec mutant, secretion of a protein itself is inhibited by high temperature, and thus, sec mutant is not suitable for secretion and production of glycoproteins.

Accordingly, since these mutants cannot completely biosynthesize the subject high mannose type sugar chain, they are considered unsuitable as host yeast for producing a mammalian type sugar chain.

Meanwhile, a pathway for sugar chain biosynthesis on endoplasmic reticulum (ER) in yeast has been revealed by isolating a mutant having some defects in various phases of tne biosynthesis and biochemically analyzing the isolated mutant. An alg (Asparagine-linked glycosylation) mutant was isolated by a skillful selection method in which mutants that avoided damage/extinction due to radiation were concentrated because the incorporation of [³H] mannose into its sugar chain was less than a wild type cell having an outer sugar chain. Among those, an alg3 mutant accumulates Dol-pp-GlcNAc₂-Man₅ (Dol-pp represents dolichol pyrophosphate) under non-permissive temperature (Tanner, W. et al., Biochim. Biophys. Acta., 906, 8199 (1987)). Also, Jigami et al. conducted an analysis using Δoch1mnn1alg3 triple mutant (Jigami et al., TanpakushitsuKakusanKoso, vol. 39, No. 4, p. 657 (1994)). A sugar chain of mannoprotein was subjected to fluorescence-labeling using PA (2-aminopyridine) and was then analyzed. As a result, main components exhibited two peaks corresponding to Man₈GlcNA₂-PA and Man₅GlcNAc₂-PA. Results of α-1,2-mannosidase digestion, FAB-MS and the like indicated that the former was identical to ER core sugar chain. In contrast, regarding the latter, α-1,2-mannosidase digestion removed two molecules of Man and produced Man₂GlcNAc₂-PA, and specific cleavage of α-1,6 linked Man (partial acetolysis) removed only one molecule of Man. These results showed that the sugar chain of Man₅GlcNAc₂-PA produced by this triple mutant had an incomplete core type sugar chain structure as shown in the above formula (II). This triple mutant produces not only Man₅GlcNAc₂ but also Man₈GlcNAc₂ because alg3 mutation accumulating Man₅GlcNAc₂-pp-Dol on dolichol pyrophosphate is leaky.

In contrast, regarding the latter, recent development in genetic engineering enabled construction of a deficient mutant in which a number of target genes have been disrupted.

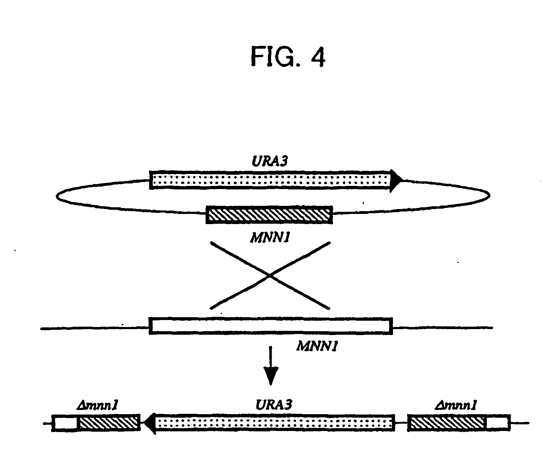

Yeast that has auxotrophic mutation is known, and auxotrophic mutation includes leu2 mutation, trp1 mutation, ura3 mutation, ade2 mutation, and his3 mutation (Yasuj i Oshima, writer and editor, Seibutsukagaku Jikkenho 39, Kobo Bunshi Idengaku Jikkenho, 119-144 (1996)). Introduction of an original gene into the mutant free from mutation can eliminate these auxotrophic properties and enable growth of the yeast without adding an essential component in a medium. Based on this principle, yeast genes can be disrupted (Fig. 4). In this method, through in vitro operation, target gene DNA on a plasmid is first fragmentated or partially deleted, and an adequate selection marker gene DNA is then inserted thereto to prepare a construct in which a selection marker is sandwiched between upstream region and downstream region of the target gene. Subsequently, the linear DNA having this structure is introduced into a yeast cell to effect two recombinations at a homologous portion between both ends of the introduced fragment and a target gene on chromosome, thereby substituting with a DNA construct in which a selection marker is sandwiched (Rothstein, Methods Enzymol., 101, 202-211 (1983)). This method requires one selection marker to disrupt one gene.

Molecular breeding of a yeast strain lacking its outer sugar chain has already been described in Japanese Patent Laid-Open Nos. Hei6-277086(277086/1994) and Hei9-266792(266792/1997). However, a glycoprotein sugar chain produced from a double mutant (Δoch1Δmnn1) described in Japanese Patent Laid-Open No. 277086/1994 was found to comprise an acidic sugar chain containing a phosphoric acid residue. This acidic sugar chain is a structure which is absent in a sugar chain derived from mammals such as humans, and it may be recognized as a foreign substance in a mammal body, and may exhibit antigenicity (Ballou, Methods Enzymol., 185, 440-470 (1990)). Hence, a quadruple mutant (described in Japanese Patent Laid-Open No. 266792/1997) was constructed in which a function of a gene positively regulating transfer of mannose-1-phosphate (MNN4) and a function of a mannose transferase gene performing an elongation reaction for an O-linked sugar chain (KRE2) are disrupted. It was shown that a sugar chain of glycoprotein produced by the yeast strain described therein had a structure of an M8 high mannose type sugar chain of interest. Further, a strain in which *Aspergillus saitoi*-derived α-1,2-mannosidase gene is introduced in this yeast mutant has a high mannose type sugar chain (Man₅₋₈GlcNAc₂) in which one to several mannose residues were cleaved (Chiba et al., J. Biol. Chem., 273, 26298-26304 (1998)).

Shimma et al. produced a different variety of quadruple mutant in which alg3 mutation was further introduced (Shimma Y. et al., Mol. Gen. Genet., 256, 469-480 (1997); Wang et al., J. Biol. Chem. 272, 18117-18124 (1997); Yoichi Shimma, Yoshifumi Jigami, Abstracts of 32nd Forum of the Yeast Genetics Society of Japan , p. 64 (1999); Shimma Y. et al., Abstracts of XIX International Conference On Yeast Genetics and Molecular Biology, p. 443 (1999)).

A sugar chain of glycoprotein produced from these yeast strains is also present in mammals, and thus does not have antigenicity. As is also apparent from the example of erythropoietin, however, a glycoprotein containing a high mannose type sugar chain may not exhibit activity equivalent to a glycoprotein produced from mammalian cells because of its sugar chain structure. Also, this quadruple mutant cannot use four selection markers (leu2, ura3, lys2, trp1) any more which have been used for recessive mutations for disruption of target genes. One of the remnant auxotrophic markers is artificially disrupted instead of mutation, and thus, incorporation in a yeast chromosome utilizing homology with these marker gene loci on the chromosome cannot be carried out. Thus, it is difficult to introduce into these yeast strains a number of genes belonging to a group of sugar chain hydrolysis enzyme genes, a group of glycosyltransferase genes, or a group of sugar nucleotide transporter genes necessary for producing a mammalian type sugar chain, or genes for producing useful glycoprotein. As described above, ten-odd groups of glycosyltransferase are necessary for producing a mammalian type sugar chain and adoption of this yeast cell as a host is considered unsuitable for performing alteration and regulation of a sugar chain structure as desired.

Chiba et al. J. Biol. Chem., vol. 273, no. 41, 9 October 1998, pp. 26298-26304 discloses mutants of Saccharomyces cerevisiae with a disruption in the genes och1, mnn1 and mnn4. It further discloses production of glycoproteins with a "hybrid" Man₅GlcNac₂-structure. The introduction of the GnT-I gene in the host cells is not described therein.

Yoshida et al, Glycobiology, vol. 9, 1999, pp. 53-58 states that GnT-I is essential for the conversion of highs mannose-type N-glycans to hybrid- and complex-type ones. In this document the GnT-I activity was confirmed only in vitro where oligosaccharides were used. This document further states that in A. nidulans the GnT-I is active *in vitro*, but does not function *in vivo.*

Nakanishi-Shindo et al., J. Biol. Chem., 268, pp. 26338-26345 (1993) discloses och1, mnn1, alg3 triple mutants of Saccharomyces cerevisiae, wherein the och1 gene is disrupted and the mnn1 and alga3 genes are mutated. The activity of the alg3 gene product still remains, since alg3 is not disrupted completely, but rather is only naturally mutated.

Verostek et al., J. Biol. Chem., vol. 268, no. 16, 5 June 1996, pp. 12104-121154 is a further document on alg3 mutants. It relates to glycoprotein biosynthesis in an alg3 Saccharomyces cerevisiae mutant.

The object of the present invention is to overcome the above problems in producing Asn-linked glycoprotein with yeast and to provide a process for producing a sugar chain having a sugar chain structure identical to that of a high mannose type, a hybrid type, and a complex type, produced by human and other mammalian cells and a glycoprotein comprising the sugar chain using yeast.

### DISCLOSURE OF THE INVENTION

We have carried out thorough studies to attain the above object, and as a result, have produced successfully a novel yeast mutant (auxotrophic triple mutant) in which, among genes biosynthesizing a yeast specific outer sugar chain, a gene coding for α-1,6 mannosyl transferase performing an initial elongation addition reaction (OCH1), a gene coding for α-1,3 mannosyl transferase adding mannose to a non-reducing end of a sugar chain (MNN1), and a gene regulating addition of mannose-1-phosphate (MNN4), have been disrupted, while retaining an auxotrophic mutation that is a selecting marker, that is, without finally introducing genes complementing an auxotrophic property. The mutant can produce a sugar chain having an identical sugar chain structure to a mammalian type sugar chain. Further, various mammalian type sugar chains can be produced by introducing genes for biosynthesis of a mammalian type sugar chain into the mutant.

Specifically, the present invention relates to the following
1. A method for preparing a yeast mutant producing a glycoprotein having a sugar chain represented by formula (IV) set forth below: 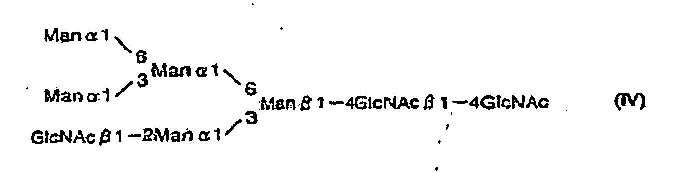 wherein Man represents mannose and GlcNAc represents N-acetylglucosamine, and wherein the method comprises the steps of:
   disrupting the MNN1 gene, MNN4 gene, and OCH1 gene, in a wild-type yeast; and
   introducing a gene encoding an α-mannosidase I with a C-terminal ER-retention signal and a GnT-1 gene, which comprises the ORF, into said yeast.
2. A method for preparing a yeast mutant,which comprises the steps of:
   disrupting the MAN1 gene, MNN4 gene, and OCH1 gene, in a wild-type yeast; and
   introducing a gene encoding an α-mannosidase I with a C-terminal ER-retention signal, a GnT-I gene, which comprises the ORF, an α-mannosidase II gene, and a GnT-II gene into said yeast.
3. A method for preparing a yeast mutant, which comprises the steps of:
   disrupting the ALG3 gene, the MNN1 gene, the MNN₄ gene, and the OCH1 gene in a wild-type yeast; and
   introducing with a C-terminal ER-retention signal an α-mannosidase I a gene encoding into said yeast.
4. The method according to claim 3, further comprising introducing a GnT-I gene, which comprises the ORF, and a GnT-II gene into said yeast.
5. The method according to any of the preceding claims, wherein the yeast mutant has at least one auxotrophic mutation trait selected from ura3 mutation, his3 mutation, leu2 mutation, ade2 mutation, trp1 mutation, and can1 mutation.
6. The method according to any of the preceding claims, wherein the yeast mutant has a ura3 mutation.
7. The method according to any of the preceding claims, wherein the α-mannosidase I gene is derived from *Aspergillus saitoi.*
8. A method for preparing a yeast mutant according to claims 1 to 7, which comprises disrupting the OCH1 gene with a uracil marker.
9. The method according to claim 8, wherein the uracil marker is ura3.
10. A method for producing a glycoprotein having a sugar chain represented by formula (IV) set forth below: 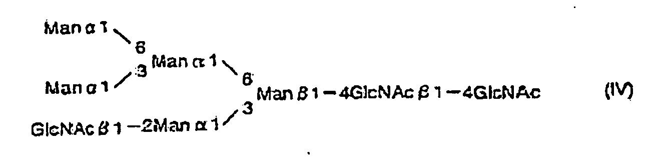 wherein Man represents mannose and GlcNAc represents N-acetylglucosamine, wherein the method comprises the steps of
   - culturing the yeast mutant produced by the method according to claim 1 in a medium,
   - producing and accumulating the glycoprotein in the culture product, and
   - collecting the glycoprotein from the culture product.
11. A mutant yeast produced by any of the methods according to claims 1 to 9.

This specification includes the contents as described in the specification and/or drawings of Japanese Patent Application No. Hei11-233215(233215/1999), which is a priority document of the present application.

### BRIEF DESCRIPTION OF DRAWINGS

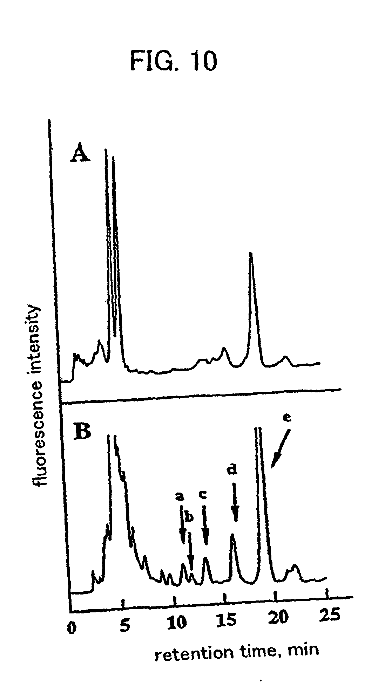

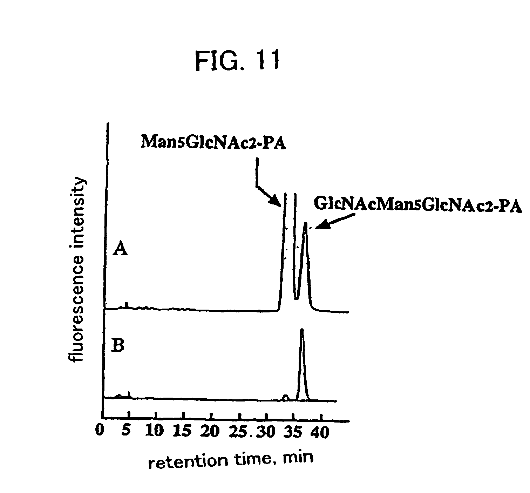

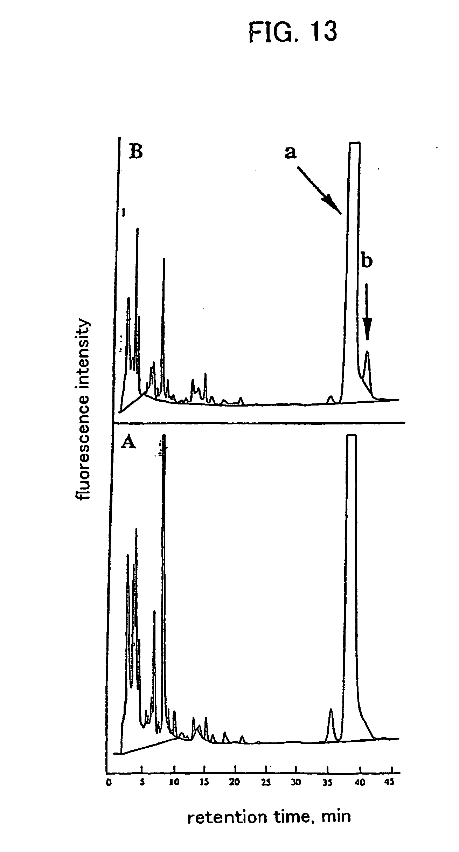

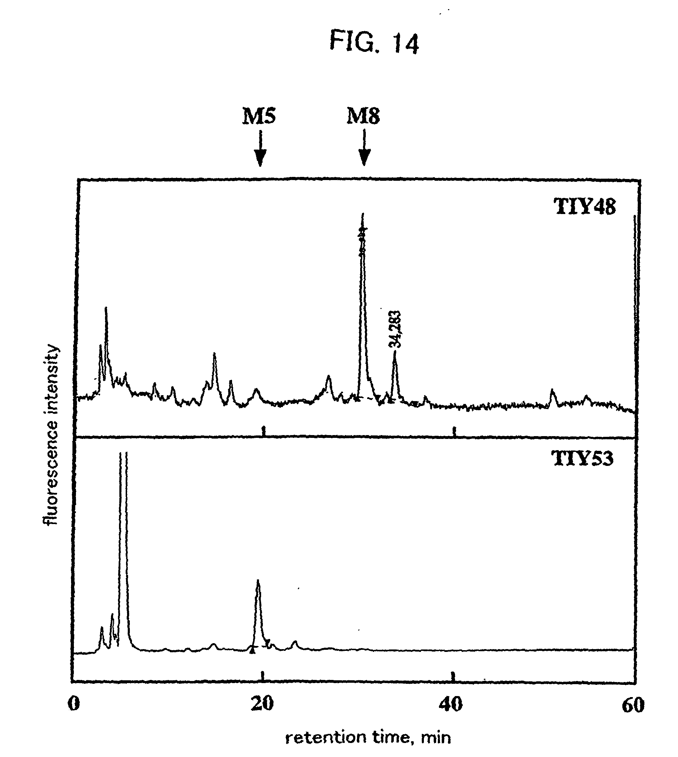

Fig. 1 shows a biosynthesis pathway for an N-linked sugar chain that is common in mammals.
Fig. 2 shows a biosynthesis pathway for an N-linked sugar chain in yeast (*S. cerevisiae*), wherein H, C, E respectively lead to I, D, F in Fig. 3.
Fig. 3 shows a biosynthesis pathway (continuation) for an N-linked sugar chain in yeast (*S*. *cerevisiae*).
Fig. 4 shows a conventional process for disrupting a yeast gene.
Fig. 5 shows a process for disrupting genes without finally introducing genes complementing an auxotrophic property.
Fig. 6 shows an analyzed structure of a sugar chain of mannoprotein on a cell surface of TIY19 strain.
Fig. 7 shows the structure, analyzed using Amide-80 column, of a sugar chain of mannoprotein on the cell surface of TIY19 strain in which α-1,2-mannosidase gene is introduced.
   a: a sugar chain of a mannan glycoprotein of TIY19 strain
   b: a sugar chain of a mannan glycoprotein of TIY19 strain in which α-1,2-mannosidase is introduced.
Fig. 8 shows the structure, analyzed using ODS-80T_{M} column, of a sugar chain of mannoprotein on the cell surface of TIY19 strain in which α-1,2-mannosidase gene is introduced.
   a: a standard sugar chain having a structure represented by formula (III)
   b: a fraction obtained in Fig. 6.
Fig. 9 shows a result of measurement of GnT-I activity.
Fig. 10 shows the structure, analyzed using Amide-80 column, of a sugar chain of mannoprotein on the cell surface of TIY19 strain in which α-1,2-mannosidase gene and GnT-I gene are introduced.
   A: analyzed structure of a sugar chain of TIY19 strain in which only a vector was introduced
   B: analyzed structure of a sugar chain of TIY19 strain in which α-1,2-mannosidase gene and GnT-I gene were introduced
      a: Man₅GlcNAc₂-PA
      b: GlcNAcMan₅GlcNAc₂-PA
      c: Man₆GlcNAc₂-PA
      d: Man₇GlcNAc₂-PA
      e: Man₈GlcNAc₂-PA
Fig. 11 shows the structure, analyzed using ODS-80T_{M} column, of a sugar chain of mannoprotein on the cell surface of TIY19 strain in which α-1,2-mannosidase gene and GnT-I gene are introduced.
   A: mixture of standard product
   B: a fraction obtained in Fig. 10.B.

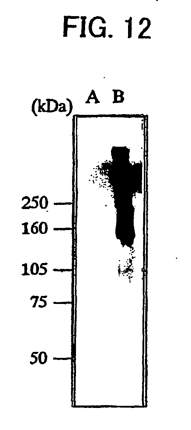

Fig. 12 shows Western blot analysis using a cell extract of YPH500 strain in which α-mannosidase II gene is introduced.
   A: Result of Western blot analysis of a cell extract from YPH500 strain in which only a vector (pYEX-BX-3HA) is introduced
   B: Result of Western blot analysis of a cell extract from YPH500 strain in which a vector (pYEOM2-HA) containing a chimeric α-mannosidase II gene is introduced
Fig. 13 shows a result of measurement on α-mannosidase II activity using a cell extract from YPH500 strain in which α-mannosidase II gene is introduced.
   A: Result of measurement on activity in YPH500 strain in which only a vector (pYEX-BX-3HA) is introduced
   B: Result of measurement on activity in YPH500 strain in which a vector (pYEOM2-HA) containing a chimeric α-mannosidase II gene is introduced
      a: GlcNAcMan₅GlcNAc₂-PA
      b: GlcNAcMan₃GlcNAc₂-PA
Fig. 14 shows a structure of FGF sugar chain in TIY 48 strain in which FGF gene is introduced (upper stage) and in TIY 53 strain in which FGF gene and α-1,2-mannosidase gene are introduced (lower stage), analyzed using Amide-80 column.

### DESCRIPTION OF SYMBOLS

- GlcNAc, GN:: N-acetylglucosamine
- Man, M :: mannose
- PA :: 2-aminopyridylation

### BRIEF DESCRIPTION OF SEQUENCE LISTING

SEQ ID NO: 1 represents primer A for amplifying 5' region in MNN1 gene.

SEQ ID NO: 2 represents primer B for amplifying 5' region in MNN1 gene.

SEQ ID NO: 3 represents primer C for amplifying 3' region in MNN1 gene.

SEQ ID NO: 4 represents primer D for amplifying 3' region in MNN1 gene.

SEQ ID NO: 5 represents primer E for amplifying 3' region in MNN4 gene.

SEQ ID NO: 6 represents primer F for amplifying 3' region in MNN4 gene.

SEQ ID NO: 7 represents primer G for amplifying 5' region in MNN4 gene.

SEQ ID NO: 8 represents primer H for amplifying 5' region in MNN4 gene.

SEQ ID NO: 9 represents primer I for amplifying 5' region in ALG3 gene.

SEQ ID NO: 10 represents primer J for amplifying 5' region in ALG3 gene.

SEQ ID NO: 11 represents primer K for amplifying 3' region in ALG3 gene.

SEQ ID NO: 12 represents primer L for amplifying 3' region in ALG3 gene.

SEQ ID NO: 13 represents primer M for amplifying N-terminal region in α-mannosidase II gene.

SEQ ID NO: 14 represents primer N for amplifying N-terminal region in α-mannosidase II gene.

SEQ ID NO: 15 represents primer O for amplifying a central region in α-mannosidase II gene.

SEQ ID NO: 16 represents primer P for amplifying a central region in α-mannosidase II gene.

SEQ ID NO: 17 represents primer Q for amplifying C-terminal region in α-mannosidase II gene.

SEQ ID NO: 18 represents primer R for amplifying C-terminal region in α-mannosidase II gene.

SEQ ID NO: 19 represents sequence S of double-stranded DNA coding for a gene for HA-tag linkage repeating three times.

SEQ ID NO: 20 represents sequence T of double-stranded DNA coding for a transmembrane region in OCH1 gene.

SEQ ID NO: 21 represents primer U for amplifying a part of a catalytic region in α-mannosidase II gene.

SEQ ID NO: 22 represents primer V for amplifying a part of a catalytic region in α-mannosidase II gene.

SEQ ID NO: 23 represents primer W for amplifying human UDP-GlcNAc transporter gene.

SEQ ID NO: 24 represents primer X for amplifying human UDP-GlcNAc transporter gene.

SEQ ID NO: 25 represents primer Y for amplifying human prepro α-factor and FGF gene.

SEQ ID NO: 26 represents primer Z for amplifying human prepro α-factor and FGF gene.

### EMBODIMENT FOR CARRYING OUT THE INVENTION

The present invention will be described in detail.

Mutation traits necessary for yeast mutant according to the present invention are mutations of outer chain biosynthetic genes specific to yeast. Specific examples are och1 mutation, mnn1 mutation, and mnn4 mutation, or och1 mutation, mnn1 mutation, mnn4 mutation, and alg3 mutation.

That is, the yeast mutant may be obtained by natural mutantion or artificial mutantion so far as it has the above mutations.

Auxotrophic mutation traits for introducing exogenous genes in the yeast mutant of the present invention are defined by the yeast strain used. Specifically, the mutation traits are selected from ura3 mutation, his3 mutation, leu2 mutation, ade2 mutation, trp1 mutation, and can1 mutation. The number of auxotrophic mutation traits depends on the number of genes to be introduced, and, one auxotrophic mutation trait is generally needed to introduce one gene. When a plurality of genes is introduced, a fragment of the introduced genes is long, introduction efficiency is lowered, and thus, expression efficiency is lowered. Thus, the more genes introduced, the greater the number of auxotrophic mutation traits required.

The phrase "genes complementing an auxotrophic property" used herein refers to genes for synthesis of body compositions such as amino acid and nucleic acid. A mutation trait contains a mutation in which these genes do not function, and thus, complementing genes are original functioning genes themselves. Hence, genes derived from the original yeast strain are preferred.

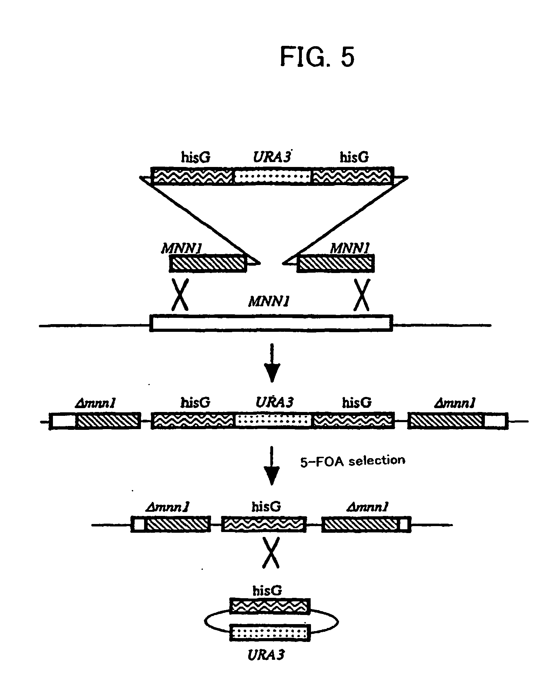

The phrase "without final introduction of genes complementing an auxotrophic property" or "without finally introducing genes complementing an auxotrophic property" refers to a phenomenon in which one or more selection markers, that is, auxotrophic mutation traits, are utilized in disruption of one or more genes (introduction of mutation trait), the number of the auxotrophic traits remaining is the same as the number of disrupted genes after disruption, and the same auxotrophic traits can be repetitiously used in another gene disruption (see Fig. 5).

The yeast mutant of the present invention in which an auxotrophic mutation trait for introducing exogenous genes is retained while yeast-specific genes for biosynthesis of outer sugar chain are disrupted (hereinafter referred to as auxotrophic mutant) can be prepared as follows.

At the outset, regarding isolation of a DNA gene fragment necessary for disruption of a target gene, its location on the chromosome is known due to a genome project on *Saccharomyces cerevisiae* (Goffeau et al., Nature, 387 (suppl.), 1-105 (1997)), and thus, distribution of gene fragments including a vicinity of the target genes are available from public institutes such as ATCC (American Type Culture Collection) in the USA (ATCC Recombinant DNA materials, 3rd edition, 1993). It is also possible to extract genome DNA from *S. cerevisiae* by a general method and select the target genes. Extraction of genome DNA from *S. cerevisiae* can be carried out in accordance with, for example, a method by Cryer et al. (Methods in Cell Biology, 12, 39-44 (1975)) and a method by P. Philippsen et al. (Methods Enzymol., 194, 169-182 (1991)).

The target genes are amplified by PCR and then subjected to gene disruption. PCR can amplify the specific DNA fragment to several hundreds of thousand times or more in about 2 to 3 hours *in vitro* using a combination of a sense antisense primers at both ends of the region, heat resistant DNA polymerase, a DNA amplification system and the like. In amplification of the target genes, 25 to 30mer synthetic single-stranded DNA is used as a primer and genome DNA is used as a template.

In the present invention, disruption of the target genes is basically performed in accordance with a method disclosed in Rothstein, Methods Enzymol., 101, 202-211 (1983). In this method, target gene DNA on a plasmid is first fragmentated or partially deleted, an adequate selection marker gene DNA is inserted therein to prepare a structure in which a selection marker is sandwiched between upstream region and downstream region of the target gene. Subsequently, this structure is introduced into a yeast cell. The above operation effects two recombinations at a homologous portion between both ends of the introduced fragment (DNA structure in which a selection marker is sandwiched) and a target gene on the chromosome, thereby substituting the target gene on the chromosome with the introduced fragment.

Specific explanation is provided using an example of preparation of MNN1 gene disrupted strain. hisG-URA3-hisG cassette is cleaved out with a restriction enzyme from a plasmid in which a hisG gene DNA fragment of Salmonella is linked to both ends of a URA3 gene constructed according to Alani et al. (Alani et al., Genetics, 116, 541-545 (1987)) and is inserted into a target gene on a plasmid to construct a disrupted allele. A gene-disrupted strain is obtained by substituting with a target gene of the chromosome using this plasmid. A URA3 gene that is inserted into a chromosome is sandwiched by hisGs and is dropped out of the chromosome together with 1 copy of hisG due to homologous recombination between hisG sequences. 1 copy of a hisG fragment still remains in a disrupted target gene on the chromosome, however, a host cell is a Ura phenotype (Fig. 5). Homologous recombination between hisGs can be carried out by means of 5-fluoroorotic acid (5-FOA). ura3 mutant is 5-FOA-resistant (Boeke et al., Mol. Gen. Genet., 197, 345-346 (1984); Boeke et al., Methods Enzymol.,154, 165-174 (1987)), and a cell strain having Ura3⁺ phenotype can no longer grow in 5-FOA medium. Thus, separation of a strain having a resistant trait in a medium to which 5-FOA is added enables operation using URA3 again.

This MNN1 gene disrupted strain is subjected to MNN4 gene disruption and OCH1 gene disruption in the same manner, whereby the subject auxotrophic triple mutant of the present invention (Δoch1Δmnn1Δmnn4) is obtained. Further ALG3 gene disruption in the same manner can provide the subject auxotrophic quadruple mutant of the present invention (Δoch1Δmnn1Δmnn4Δalg3).

In an "artificially disrupted strain" in which artificial gene disruption is carried out in the above described manner, therefore, an auxotrophic mutation trait of the original yeast strain is not damaged by the gene disrupting operation. Thus, the number of the auxotrophic mutation traits of the artificially disrupted strain is equivalent to the number of auxotrophic mutation traits of the original yeast strain, i.e., at least one, even though it is a triple mutant or quadruple mutant.

On the other hand, in a "natural mutant" in which gene disruption is spontaneously effected without using the above method, the increase and decrease of the number of auxotrophic mutation traits is not related since the above method is not utilized.

Regarding preparation of a yeast mutant producing M8 high mannose type sugar chain according to the present invention, when prepared in accordance with a conventional method using a yeast strain having six auxotrophic mutation traits in order to disrupt OCH1, MNN1, and MNN4 genes, only three auxotrophic mutation traits remain, and thus, the number of auxotrophic mutation traits of the mutant is at least four.

When a yeast mutant producing M8 high mannose type sugar chain in which mutation has occurred in OCH1, MNN1, and MNN4 genes and further in ALG3 gene is produced, natural mutants having mnn1 mutation and alg3 mutation can be utilized in production according to the conventional method, however, OCH1 and MNN4 genes should be further disrupted. Thus, two auxotrophic mutation traits are utilized. Hence, use of the above yeast strain having six auxotrophic mutation traits results in only four remaining auxotrophic mutation traits. Thus, the number of the auxotrophic mutation traits of the mutant is at least five.

In the above operation, markers imparting resistance against agents such as G418, cerulenin, Aureobasidin, Zeocin, canavanine, cycloheximide, and tetracycline may be used as the selection marker in addition to auxotrophic markers such as URA3. Adoption of genes imparting, for example, solvent resistance against methanol, ethanol and the like, osmotic pressure resistance against glycerol, salt and the like, and resistance to metal ions such as copper as a marker can introduce and disrupt genes.

When a general method, for example, use of a phage as a vector, is utilized as a method for introducing DNA into a cell and transforming through this in the above operation, DNA can be efficiently incorporated into a host by a method for infecting phage to Escherichia coli host and the like. Methods for transforming yeast using a plasmid include a method in which a plasmid is incorporated by treating with lithium salt to facilitate spontaneous DNA incorporation and a method for electrically introducing DNA into a cell (Becker and Guarente, Methods Enzymol., 194, 182-187 (1991)).

In the above operation, isolation, purification and the like of DNA can be carried out by a conventional method, for example, in the case of *Escherichia coli*, DNA extraction by alkali/SDS technique and ethanol precipitation, and further DNA purification by RNase treatment, PEG precipitation and the like. Determination of the DNA sequence of the genes can also be carried out by a conventional method, for example, a dideoxy method (Sanger et al., Proc. Natl. Acad. Sci., USA, 74, 5463-5467 (1977)). Further, determination of the above DNA nucleotide sequence can be easily carried out using a commercially available sequencing kit and the like.

The auxotrophic mutant thus prepared can produce mammalian high mannose type sugar chain. In order to produce a mammalian type sugar chain of hybrid type and complex type, a group of yeast-specific sugar chain hydrolase genes and a group of yeast-specific glycosyltransferase genes are further introduced into the mutant. As described above, a sugar chain is originally biosynthesized in ER and in Golgi apparatus, and thus, sugar nucleotides as a starting materials for a sugar chain should be present in these organs. However, transporters for these sugar nucleotides are absent in the yeast, or even if present, only a very small amount thereof is present in organs where a sugar chain is actually biosynthesized. Thus, a group of sugar nucleotide transporter genes, which transport sugar nucleotides biosynthesized in cytoplasm from cytoplasm to ER and Golgi apparatus, is further necessary.

In the present invention, therefore, genes belonging to the group of sugar chain hydrolase genes, the group of glycosyltransferase genes, and the group of sugar nucleotide transporter genes are referred to as "genes for biosynthesis of a mammalian type sugar chain".

The group of sugar chain hydrolase genes include genes for α-mannosidase (α-mannosidase I, α-mannosidase II), the group of glycosyltransferase genes include genes for N-acetylglucosaminyl transferase (GnT-I, GnT-II, GnT-III, GnT-IV, GnT-V), galactosyl transferase (GalT), fucosyl transferase (FucT) and the like, and the group of sugar nucleotide transporter genes include genes for UDP-GlcNAc transporter, UDP-Gal transporter and the like. These genes may be isolated mammalian-derived genes or may be synthesized genes.

Regarding the "genes for biosynthesis of a mammalian type sugar chain", genes belonging to one, or two or more groups of the above-mentioned genes are introduced in the number necessary for producing the subject sugar chain. When a plurality of genes is to be introduced, these genes may belong to the same group or may belong to different groups.

When the auxotrophic mutant or a mutant in which the group of exogenous genes are introduced in the auxotrophic mutant is cultured in a medium, an outer sugar chain content specific to yeast decreases, and a glycoprotein containing Asn-linked sugar chain identical to a high mannose type sugar chain (Man₅₋₈GlcNAc₂), a hybrid type sugar chain (GlcNAcMan₅GlcNAc₂), and a complex type sugar chain (Gal₂GlcNAc₂Man₃GlcNAC₂ and the like), produced from mammalian cells, can be produced within a yeast cell or outside the cell.

More specifically, when a triple mutant (Δoch1Δmnn1Δmnn4) is used as an auxotrophic mutant, a hybrid type sugar chain may be produced by introducing α-mannosidase I gene and GnT-I gene into the mutant. Also, introduction of genes for biosynthesis of a mammalian type sugar chain (α-mannosidase II, GnT-II, GalT, UDP-GlcNAc transporter, UDP-Gal transporter genes) can produce a biantenary complex type sugar chain (Gal₂GlcNAc₂Man₂GlcNAc₂).

Introduction of GnT-IV and GnT-V genes can produce a triantenary complex type sugar chain and a tetraantenary complex type sugar chain.

When a quadruple mutant (Δoch1Δmnn1Δmnn4Δalg3) is used as an auxotrophic mutant, without introducing α-mannosidase II gene, a biantenary complex sugar chain (Gal₂GlcNAc₂Man₂GlcNAc₂) can be produced by introducing a genes for biosynthesis of a mammalian type sugar chain (α-mannosidase I, GnT-I, GnT-II, Gal-T, UDP-GlcNAc transporter, UDP-Gal transporter genes).

In order to provide the sugar chain and the glycoprotein with a high yield, the enzyme is preferably subjected to high level expression in an adequate organ (for example Golgi apparatus) . Thus, use of genes conforming to the frequency of codon usage in yeast is effective. To localize the enzyme in adequate organs, addition of signal sequence and the like of yeast is effective. In introducing genes, a method may be considered wherein vectors such as 2 µm plasmid type (YEp type), a chromosome incorporated type (YIp type) and the like are used, and a method to be adopted can be determined depending on purposes. YEp type vector can introduce many copies of genes, and thus, genes can be expressed in a large quantity. On the other hand, YIp type vectors can render genes to be present in a chromosome, and thus, the genes can be stably retained. Promoters necessary for expressing a gene include constitutive expression promoters such as GAPDH, PGK and the like and inducible expression promoters such as GAL1, CUP1 and the like without particular limitation. In producing a sugar chain, a constitutive expression promoter is preferred. However, when one or a plurality of a sugar chain hydrolase, a glycosyltransferase, and a sugar nucleotide transporter genes are to be expressed, it may affect propagation of yeast. Therefore, in this case, use of an inducible promoter and the order of introduction of genes should be considered.

The auxotrophic mutant of the present invention includes mutants obtained by a drug, ultraviolet irradiation, and natural mutation in addition to mutants obtained by the above artificial gene disruption. This natural type mutant can also produce a mammalian type sugar chain or a glycoprotein having a mammalian type sugar chain by introducing the above genes for biosynthesis of a mammalian type sugar chain (a group of sugar chain hydrolase genes, a group of glycosyltransferase genes, and genes belonging to a group of sugar nucleotide transporters).

In order to produce a glycoprotein that has the above sugar chain and is derived from different species, a gene is provided that is connected to downstream of a promoter capable of expressing a gene (cDNA and the like) coding for the subject glycoprotein in yeast by adopting the yeast mutant as a host, and then incorporated into the yeast host by homologous recombination. Alternatively, the gene may be inserted into a plasmid and used for transforming the host to prepare a transformant. The subject glycoprotein, produced within the yeast cell or outside the cell, may be recovered by culturing the transformant in accordance with the known method.

The yeast mutant can be cultured in accordance with a conventional method that is commonly utilized in culturing yeast. For example, a synthetic medium (including carbon source, nitrogen source, inorganic salts, amino acids, and vitamins) to which various medium components supplied from Difco are added and from which an amino acid, which is rendered capable of supply by a marker necessary for duplication and retention of a plasmid, is removed may be used (Sherman, Methods Enzymol., 194, 3-57 (1991)).

In order to isolate and purify a glycoprotein from the cultured product (culture solution, cultured cells), a conventional method for isolating and purifying proteins may be used.

For example, after completion of culturing, cells are recovered by centrifugation, and suspended in an aqueous buffer. Cells are then disrupted by an ultrasonic grinder, French press, Menton Gaulin homogenizer, DYNO-Mill and the like to obtain a cell-free extract. From the supernatant prepared by centrifugation of the cell-free extract, a purified preparation can be obtained using conventional methods for isolating and purifying proteins, that is, solvent extraction, salting-out using ammonium sulfate and the like, desalting, a precipitation method using an organic solvent, anion exchange chromatography using resins such as diethylaminoethyl (DEAE)-sepharose, cation exchange chromatography using resins such as 5-Sepharose FF (Pharmacia), hydrophobic chromatography using resins such as butyl sepharose and phenyl sepharose, gel filtration using a molecular sieve, affinity chromatography using resins such as His Bind resin (Novagen), chromatofocusing, and electrophoresis such as isoelectric focusing phoresis alone or in combination of two or more.

### EXAMPLES

The present invention will be described in more detail with reference to the following examples, however, these examples do not limit the technical scope of the present invention.

### [Example 1] Breeding of yeast mutant (Δmnn1Δmnn4Δoch1 auxotrophic triple mutant) capable of producing a mammalian type sugar chain

### (1) Preparation of Δmnn1 auxotrophic mutant and properties thereof

A cassette (HUH) in which *Salmonella* hisG gene was linked to both ends of URA3 gene by direct repeat was cleaved at BglII and BamHI from pNK51, which has been already reported (Alani et al., Genetics, 116, 541-545 (1987)), and was inserted into BamHI site in *Escherichia coli* plasmid pSP73. This plasmid was designated as pSP73-HUH.

MNN1 gene is located in the vicinity of No. 5 chromosome centromere of yeast, and the DNA nucleotide sequence of MNN1 gene is registered in the GenBank database under the accession NO. L23753 (Yip et al., Proc. Natl. Acad. Sci. USA, 9, 2723-2727 (1994)). 5' region in MNN1 gene was amplified by PCR using primer A (GGATCCGAAGAAAACCTAATACATTGAAGT: SEQ ID NO: 1) and primer B (GCATGCCCTTTGGTTTAATATAAATCTCCGGAGTGC: SEQ ID NO: 2), and 3' region was amplified by PCR using primer C (GCATGCTACATAACTCCAATCAGCAGCAAATATGTC: SEQ ID NO: 3) and primer D (GCGGCCGCGTGTTCTGTTCGGGTAACGTTTAAACCAAT: SEQ ID NO: 4). The obtained DNA fragments were incorporated into SphI site in plasmid pHYH containing HIS3 marker to prepare pHYHΔmnn1. In order to disrupt MNN1 gene using HUH cassette, 1.8 kb SphI fragment was obtained from pHYHΔmnn1, which was inserted into SphI site of pSP73-HUH to construct pSP73-Amnnl::HUH. This plasmid was cleaved at NotI site to linearize, and a wild type strain W303-1A(MATa leu2-3, 112his3-11, 15 ade2-1 ura3-1 trp1-1 can1-100) was transformed with the linear plasmid using the lithium acetate method (Ito et al., J. Bacteriol., 153, 163-168 (1983)). After transformation, the yeast cells were spread on a plate containing SD-Ura medium (2% glucose, 0.67% Yeast Nitrogen Base w/o amino acids (Difco), a mixture of nucleobases excluding uracil and amino acids (20-400 mg/L)) and cultured at 30°C for 2 days to obtain a transformant.

Genome DNA was prepared from the transformant and confirmed the incorporation of a uracil marker into a chromosome in MNN1 region by PCR. This transformant was designated as TIY1 strain.

From this strain, selection was carried out in YSD medium (1% yeast extract, 2% glucose, adenine (40 mg/L), uracil (20 mg/L)) containing 5-FOA, and URA3 gene deficient strain was obtained. In the same manner as described above, mnn1 disrupted strain lacking URA3 gene was confirmed by PCR. This strain containing Δmnn1::hisG was designated as TIY3 strain.

In the case of MNN1 disrupted strain, mobility of an invertase that is subjected to N-linked modification is known to be faster than that of a wild type strain, since it lacks α-1,3 linked mannose at the non-reducing terminus. A wild type strain and TIY3 strain, which were cultured in YPAD medium, were respectively resuspended in a nutrient medium (1% yeast extract, 2% Bacto peptone, adenine (40 mg/L)) containing 0.2% of sucrose and cultured for 3 hours. After collecting, the cells were suspended in SDS sample buffer and crushed with glass beads. A supernatant was then used to perform 6% SDS polyacrylamide electrophoresis. The invertase was detected by inducing with sucrose and then performing activity staining using triphenyltetrazolium (Ballou, Methods Enzymol., 185, 440-470 (1990)). As a result, it was confirmed that the mobility of the invertase produced by TIY3 strain was faster than that of the wild type strain.

### (2) Preparation of Δmnn1Δmnn4 auxotrophic double mutant and properties thereof

MNN4 gene is located on No. 11 chromosome of yeast, and the DNA nucleotide sequence of MNN4 gene is registered in the GenBank database under accession NO. D83006 (Odani et al., Glycobiology, 6, 805-810 (1996)). 3' region in MNN4 gene was amplified by PCR using primer E (AGATGCATACTAGTGGGCCCATTGTGATTGGAAT: SEQ ID NO: 5) and primer F (CCCCCGAATTCGTGTGAAGGAATAGTGACG: SEQ ID NO: 6), and 5' region was amplified by PCR using primer G (CCCCCGAATTCAAGTCGGAGAACCTGACTG: SEQ ID NO: 7) and primer H (ATGGGCCCACTAGTATGCATCTCGCGTGGCATGG: SEQ ID NO: 8). The obtained DNA fragments were incorporated into EcoRI site in the pSP73-HUH containing HUH cassette to prepare pSP73-Δmnn4::HUH. This plasmid was cleaved at SpeI site to linearize, and TIY3 strain was transformed with the linear plasmid using the lithium acetate method. After transformation, the yeast cells were spread on a plate containing SD-Ura medium and cultured at 30°C for 2 days to obtain a transformant.

Genome DNA was prepared from the transformant and confirmed the incorporation of a uracil marker into a chromosome into MNN4 region by PCR. This transformant was designated as TIY9 strain.

From this strain, selection was carried out in YSD medium containing 5-FOA and URA3 gene deficient strain was obtained. In the same manner as described above, mnn4 disrupted strain lacking URA3 gene was confirmed by PCR. This strain containing Δmnn1::hisG Δmnn4::hisG was designated as TIY11 strain.

The presence or absence of a phosphate group in a sugar chain can be determined by staining with alcian blue. Alcian blue is a positively charged dye which binds to a negative charge. When yeast cells are suspended in a buffer with a pH value of 3 and 0.1% alcian blue 8GX (Sigma, code No. A3157) is added thereto, only those cells having a phosphate group in the sugar chain are stained blue while those having no phosphate group become white. Since the sugar chain on the cell surface does not substantially have a phosphate group, Δmnn4 disrupted strain is not stained with alcian blue. A wild type strain, TIY3, and TIY11 were cultured in a nutrient medium and each cell was stained with alcian blue. As a result, it was confirmed that only TIY11 was not stained blue.

### (3) Preparation of Δmnn1Δmnn4Δoch1 auxotrophic triple mutant and properties thereof

OCH1 gene is located on No. 7 chromosome of yeast, and the DNA nucleotide sequence of OCH1 gene is registered in the GenBank database under accession NO. D11095 (Nakayama et al., EMBO J., 11, 2511-2519 (1992)). AatII-HpaI site in OCH1 gene of pBL-OCH1 which had been already constructed (Nakayama et al., EMBO J., 11, 2511-2519 (1992)) containing the full length of OCH1 gene was cleaved, and into which HUH cassette obtained from bluntended pNKY51 was inserted to prepare pBL-Δoch1::HUH. This plasmid was cleaved at SalI and BamHI to cleave out a region containing Δoch1::HUH, and TIY11 was transformed with the region using the lithium acetate method. Strains containing Δoch1 disruption exhibit sensitivity to low osmotic pressure, therefore, after transformation, transformed cells were spread on the plate containing SD-Ura medium containing 0.3 M KCl, and cultured at 30°C for 2 days to obtain a transformant.

Genome DNA was prepared from the transformant and confirmed the incorporation of a uracil marker into a chromosome in OCH1 region by PCR. This transformant was designated as TIY17 strain.

From this strain, selection was carried out in YSD medium containing 5-FOA and 0.3 M KCl and URA3 gene deficient strain was obtained. In the same manner as described above, och1 disrupted strain lacking URA3 gene was confirmed by PCR. This strain containing Δmnn1::hisG Δmnn9::hisG Δochl::hisG was designated as TIY19 strain.

This auxotrophic triple mutant TIY19 strain has been deposited internationally at the NATIONAL INSTITUTE OF BIOSCIENCE AND HUMAN-TECHNOLOGY (1-1-3, Higashi, Tsukuba, Ibaraki) as of July 27, 1999 under the accession number FERM BP-6802.

This TIY19 strain containing och1 disruption forms a high mannose type sugar chain, and thus, the mobility of invertase is known to be faster than those of a wild type strain, TIY3 strain and TIY11 strain. In order to confirm the effect of och1 disrupted strain on a sugar chain length, invertase was detected from the wild type strain, TIY3 strain, TIY11 strain, and TIY19 strain, respectively, in the manner as described above. As a result, it was confirmed that the mobility of the wild type strain, TIY3 strain, TIY11 strain, and TIY19 strain became faster in that order.

### [Example 2] Preparation of yeast mutant (Δmnn1Δmnn4Δoch1Δalg3 auxotrophic quadruple mutant) capable of producing a mammalian type sugar chain and properties thereof

ALG3 gene is located on No. 2 chromosome of yeast, and the DNA nucleotide sequence of ALG3 gene is registered in the GenBank database under accession NO. Z35844 (Feldmann et al., EMBO J., 13, 5795-5809 (1994)). 5' region in ALG3 gene was amplified by PCR using primer I (GCGGCCGCGAGACCTGAATCTTCGACACGCAAGAAAAA: SEQ ID NO: 9) and primer J (GAATTCGCTTTCGAACAAAATCAAAAGGGGCATAAC: SEQ ID NO: 10), and 3' region was amplified by PCR using primer K (GAATTCCTATCCACCAAACTCACAAGCAAGCA: SEQ ID NO: 11) and primer L (GCGGCCGCCGAGAGGGTGAACGGTGCTAACTCAGGATT: SEQ ID NO: 12). The obtained DNA fragments were incorporated into EcoRI site of pSP73-HUH containing HUH cassette to prepare pSP73-alg3::HUH. This plasmid was cleaved at NotI site to be linearized, and TIY19 strain was transformed with the linear plasmid using the lithium acetate method. After transformation, the yeast cells were spread on a plate of SD-Ura medium and cultured at 30°C for 2 days to obtain a transformant.

Genome DNA was prepared from the transformant and confirmed the incorporation of a uracil marker into a chromosome into ALG3 region by PCR. This transfomant was designated as YS134 strain.

From this strain, selection was carried out in SD medium containing 5-FOA and URA3 gene deficient strain was obtained. In the same manner as described above, alg3 disrupted strain lacking URA3 gene was confirmed by PCR. This strain containing Δmnn1::hisG Δmnn4::hisG Δoch1::hisG Δalg3::hisG was designated as YS134-4A strain.

This auxotrophic quadruple mutant YS134-4A strain has been deposited internationally at the NATIONAL INSTITUTE OF BIOSCIENCE AND HUMAN-TECHNOLOGY (1-1-3, Higashi, Tsukuba, Ibaraki) as of July 27, 1999 under the accession number FERM BP-6801.

This YS134-4A strain containing alg3 disruption forms a short sugar chain, and thus, the mobility of invertase is known to be faster than those of a wild type strain, TIY3 strain, TIY11 strain, and TIY19 strain. In order to confirm the effect of alg3 disrupted strain on a sugar chain length, invertase was detected from the wild type strain, TIY3 strain, TIY11 strain, TIY19 strain, and YS134-4A strain, respectively, in the manner as described in Example 1 (1). As a result, it was confirmed that the mobility of the wild type strain, TIY3 strain, TIY11 strain, TIY19 strain, and YS134-4A strain became faster in that order.

### [Example 3] Separation of mannoprotein on cell surface from Δmnn1Δmnn4Δoch1 auxotrophic triple mutant and analysis of sugar chain structure contained therein

Concanavalin A is a lectin exhibiting affinity to a sugar chain containing two or more residues of α-D-Man in which hydroxyl groups at positions C-3, C-4, and C-6 are unsubstituted. Immobilization of concanavalin A on a column enables separation of mannoprotein from yeast cell wall polysaccharides such as glucan or chitin. Mannoproteinon the cell surface was first separated from cells of TIY19 strain (Peat et al., J. Chem. Soc., 29 (1961)).

50 ml of YPAD medium containing 0.3 M KCl was placed in a 500-ml Sakaguchi flask and TIY 19 strain was cultured at 30°C for 24 hours. Cells were collected by centrifugation, and suspended in 10 ml of 100 mM sodium citrate buffer (pH 7.0) and heated in an autoclave at 121°C for 1 hour. After cooling, centrifugation was performed, and a supernatant was collected. Another 10 ml of water was added to the remaining solid substance and the mixture was heated and centrifuged to collect a supernatant in the same manner as described above. All the extracts were combined and added to three-times volume of ethanol. The resultant white precipitate was dried and then dissolved in a buffer for Concanavalin A (ConA) column (0.1 M sodium phosphate buffer (pH 7.2) containing 0.15 M sodium chloride and 0.5 mM calcium chloride). The solution was loaded on ConA-agarose column (0.6 x 2 cm, HONEN CORPORATION) . After washing with a buffer for ConA column, elution was performed using a buffer for ConA column containing 0.2 M α-methyl mannoside. The resultant fraction was dialyzed and freeze-dried, thereby obtaining mannoprotein.

Subsequently, the resultant mannoproteinwas treated with an enzyme to cleave out an Asn-linked sugar chain. That is, a freeze-dried sample was dissolved in 100 µl buffer for N-glycosidase F (0.1 M Tris-HCl buffer (pH 8.0) containing 0.5% SDS and 0.35% 2-mercaptoethanol) and boiled for 5 minutes. The product was cooled to room temperature, 50 µl of 7.5% Nonidet P-40, 138 µl of H₂O, and 12 µl of N-glycosidase F (Boehringer) were then added thereto, and the mixture was treated at 37°C for 16 hours. After desalting with BioRad AG501-X8 column, an equivalent volume of phenol : chloroform (1 : 1) was added and was vigorously shaken, thereby removing a surfactant and protein. Thus, a sugar chain preparation was obtained.

The obtained sugar chain was subjected to the following operation in order to perform fluorescent labeling (pyridylamination, referred to as PA ("pyridylamination" is hereinafter abbreviated to "PA")). The sugar chain preparation was concentrated to dryness, and 40 µl of a coupling reagent (a solution of 552 mg of 2-aminopyridine in 200 µl of acetic acid) was then added thereto. The mixture was then hermetically sealed and treated at 90°C for 60 minutes. After cooling to room temperature, 140 µl of a reducing reagent (a solution of 200 mg of borane-dimethylamine complex in 50 µl of H₂O and 80 µl of acetic acid) was added. The mixture was then hermetically sealed and treated at 80°C for 80 minutes. After the reaction, 200 ml of aqueous ammonia was added to the reaction mixture and an equivalent volume of phenol : chloroform (1 : 1) was further added thereto. After the mixture was vigorously shaken, an aqueous layer containing PA-oligosaccharide was collected. This procedure was repeated seven times and unreacted 2-aminopyridine was removed. A supernatant was filtered through a 0.22 µm filter, and thus, a PA-oligosaccharide preparation was obtained.

HPLC using an amide column can separate PA-oligosaccharide depending on its chain length. TSKGel Amide-80 (4.6 x 250 mm, Tosoh) was used as a column. A mixed solution of 200 mM acetic acid-triethylamine buffer (pH 7.0) and acetonitrile (35 : 65, solvent A) and a mixed solution of 200 mM acetic acid-triethylamine buffer (pH 7.0) and acetonitrile (50 : 50, solvent B) were prepared as solvents.

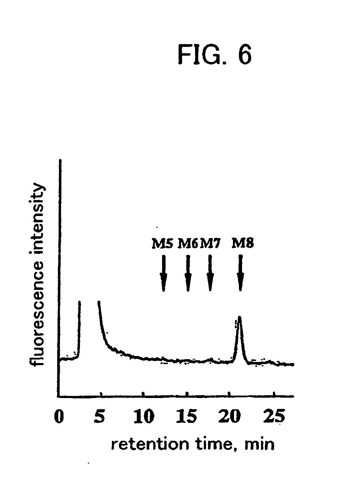

The column was equilibrated by running solvent A beforehand at a flow rate of 1.0 ml/min. Immediately after the sample injection, the ratio of solvent B was linearly increased to 50% over a period of 25 minutes. Solvent A and Solvent B were then flowed for 5 minutes remaining unchanged at 50 : 50, thereby eluting PA-oligosaccharide. The result is shown in Fig. 6. The sugar chain of mannoprotein produced from Δoch1Δmnn1Δmnn4 auxotrophic triple mutant TIY19 strain exhibited primarily one peak with an amide column. This peak was corresponding to the elution position of Man₈GlcNAc₂-PA standard (TAKARA SHUZO CO., LTD.). This indicates that a high mannose type sugar chain of Man₈GlcNAc₂ was added to mannoprotein produced from TIY19 strain.

### [Example 4] Introduction of α-mannosidase I gene into Δoch1Δmnn1Δmnn4 auxotrophic triple mutant

In order to biosynthesize a sugar chain having a structure closer to that of the mammalian-type in yeast, α-mannosidase I (α-1,2-mannosidase) gene, which controls an initial reaction thereof, may be introduced into an auxotrophic triple mutant to express. The introduced strain can biosynthesize Man₅GlcNAc₂ sugar chain that is a precursor of a mammalian hybrid type or mammalian complex type in which a high mannose type sugar chain is further shortened.

An ER-retrieval type expression plasmid pGAMH1 for α-1,2-mannosidase derived from *Aspergillus saitoi*, which has been already reported to have expression ability (Chiba et al., J. Biol. Chem., 273, 26298-26304 (1998)) was used to transform TIY19 strain by the lithium acetate method. As a control, a transformant prepared only from a vector pG3 containing no α-1,2-mannosidase gene was used. After transformation, the cells were spread on the plate containing a SD-Trp medium (2% glucose, 0.67% Yeast Nitrogen Base w/o amino acids (Difco), a mixture of nucleobases and amino acids excluding tryptophan (20-400 mg/L)) and cultured at 30°C for 2 days to obtain the transformant. The resultant transformant was designated as TIY19pGAMH1.

As with Example 3, a sugar chain was prepared from the resultant transformant to perform HPLC analysis.

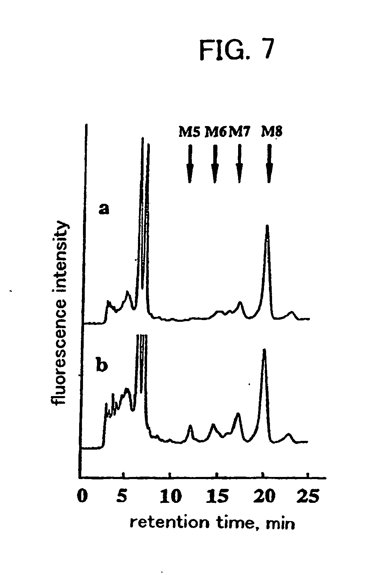

The results of analysis by the amide column are shown in Fig. 7. A sample transformed by a control vector only exhibited primarily one peak as with the results in Example 3 (Fig. 7, a), which was corresponding to the elution position of Man₈GlcNAc₂-PA standard (TAKARA SHUZO CO., LTD.). On the other hand, TIY19pGAMH1 containing α-1,2-mannosidase gene exhibited primarily four peaks (Fig. 7, b). These peaks were corresponding to the elution positions of Man₅GlcNAc₂-PA, Man₆GlcNAc₂-PA, Man₇GlcNAc₂-PA, and Man₈BGlcNAc₂-PA standards, in the order of the elution speed, i.e., from fastest to slowest. These sugar chains are referred to as a human high mannose type sugar chain.

Subsequently, Man₅GlcNAc₂-PA fraction with the fastest elution was collected and subjected to a reversed phase column.

HPLC using a reversed phase column enables separation of PA-oligosaccharide depending on its structure. TSKGel ODS-80T_{M} (4.6 x 150 mm, Tosoh) was used as a column, and 100 mM ammonium acetate buffer (pH 4.0, solvent A) and 100 mM ammonium acetate buffer containing 0.5% 1-butanol (pH 4.0, solvent B) were prepared as solvents.

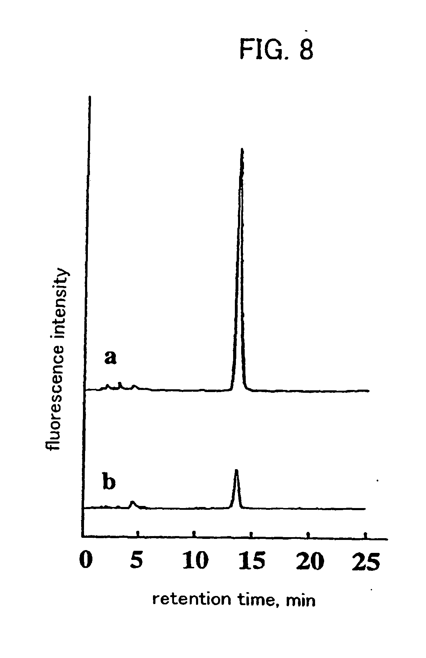

The column was equilibrated by running a mixture of solvent A and solvent B (95 : 5) beforehand at a flow rate of 1.2 ml/min. Immediately after the sample injection, the ratio of solvent B was linearly increased to 50% over a period of 20 minutes, thereby eluting PA-oligosaccharide. The result is shown in Fig. 8. The collected sugar chain fraction primarily exhibited one peak with a reversed phase column (Fig. 8, a) and this peak was corresponding to the elution position of Man₅GlcNAc₂-PA standard (TAKARA SHUZO CO., LTD.) having a structure represented by formula (III): 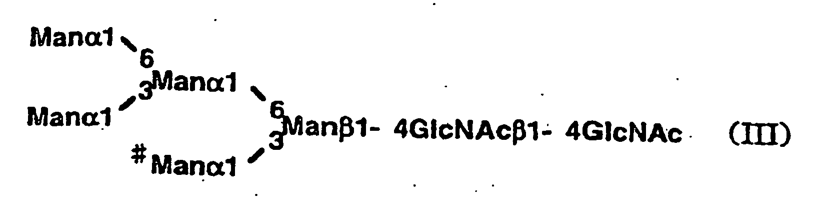 wherein Man represents mannose, GlcNAc represents N-acetylglucosamine, and # represents a site where GnT-I acts (Fig. 8, b). This indicated that mannoprotein produced from TIY19pGAMH1 strain contained Man₅GlcNAc₂ sugar chain that is a precursor of a hybrid type and a complex type.

### [Example 5] Synthesis of a hybrid type sugar chain (GlcNAcMan₅GlcNAc₂) standard

At the outset, for the purpose of confirming and examining biosynthesis of a hybrid type sugar chain (GlcNAcMan₅GlcNAc₂), GnT-I enzyme reaction was carried out in vitro to synthesize the subject sugar chain. The substrate specificity of GnT-I is very strict, and GnT-I is known to transfer GlcNAc only to mannose residue at position # through β-1,2-linkage for a sugar chain structure represented by formula (III).

Expression of rat GnT-I gene in yeast has been accomplished by Yoshida et al. (Yoshida et al., Glycobiology, 9, 53-58 (1999)). This gene was linked to downstream of GAP-DH promoter of pG3 that is a multicopy plasmid, then cleaved at SmaI - NaeI, and a region containing a promoter, ORF of GnT-I subsequent to the promoter and a terminator was cleaved out. This fragment was then introduced into SmaI site of a multicopy plasmid pYO354. This plasmid was designated as pYOG4. Using this plasmid, a wild type yeast YPH500 strain was transformed by the lithium acetate method. After transformation, the cells were spread on the plate containing a SD-Trp medium (2% glucose, 0.67% Yeast Nitrogen Base w/o amino acids (Difco), a mixture of nucleobases and amino acids excluding tryptophan (20-400 mg/L)) and cultured at 30°C for 2 days to obtain a transformant. The resultant transformant was designated as YPH500/pYOG4.

The transformant was subjected to liquid cultivation in 500 ml SD-Trp (2% glucose, 0.67% Yeast Nitrogen Base w/o amino acids (Difco), a mixture of nucleobases and amino acids excluding tryptophan (20-400 mg/L)) solution and was harvested. After washing with cold water, the cells were suspended in 5.7 ml of Spheroplast medium (50 mM potassium phosphate containing 1 M sorbitol (pH 7.5)). 9 µl of 2-mercaptoethanol and 12 mg of Zymolyase 100T were dissolved in 300 µl of Spheroplast medium and added to the suspension. The resultant mixture was heated at 30°C for 45 minutes. 1 M sorbitol (15 ml) was added and the mixture was centrifuged. Thereafter, the precipitate was washed with 15 ml of 1 M sorbitol again and harvested. To this precipitate, 4 ml of lysis buffer (10 mM triethanolamine (pH 7.2) solution containing 250 mM sorbitol, 2 µg/ml antipain, 2 µg/ml chymostatin, 3 µg/ml leupeptin, 3 µg/ml pepstatin, 1 mM benzamidine, 1 mM EDTA, 1 mM EGTA, and 1 mM PMSF) was added. Cells were destroyed by a homogenizer and centrifuged at 220 x g to recover a supernatant. This supernatant was further centrifuged at 100,000 x g and the precipitated fraction was suspended in 150 µl of lysis buffer. Thus, a GnT-I enzyme solution was obtained. Other GnT activity was not found in this standard.

The subject sugar chain was then synthesized. PA-labeled Man₅GlcNAc₂ sugar chain (purchased from TAKARA SHUZO CO. , LTD.) was used as a acceptor substrate, and placed into a tube at the amount of 200 pmol each. The contents of the tube were evaporated to dryness, and then 8.2 µl of GnT-I enzyme solution prepared above, 2 µl of 0.2 M MnCl₂, and 9.8 µl of GnT-I reaction buffer (0.17 M MES (pH 6.0), 1.7% Triton X-100, 0.34% Bovine Serum Albumin, 8.47 mM AMP, 1.69 mM UDP-GlcNAc, and 169 mM GlcNAc) were added in the tube, and incubated at 37°C for 3 hours. The reaction was terminated by 5 minutes of boiling. The reaction mixture was then filtered through a 0.22 µm filter to be subjected to HPLC.

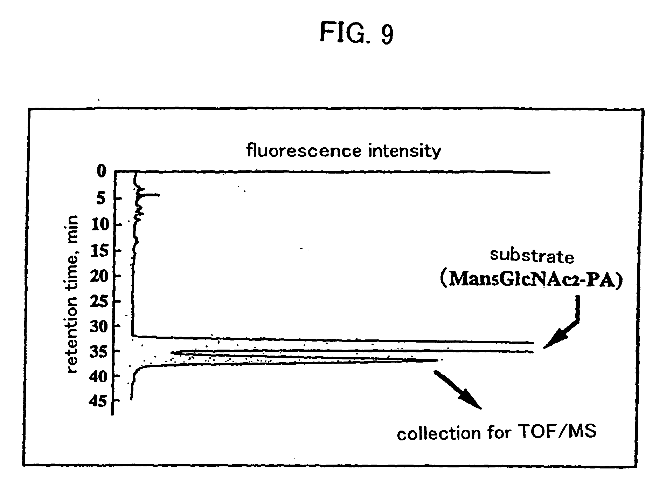

TSKGel ODS-80T_{M} (4.6 x 250 mm, Tosoh) was used as a column, and 100 mM ammonium acetate buffer (pH 6.0) containing 0.15% 1-butanol was used as a solvent. The column was equilibrated by running the solvent beforehand at a flow rate of 1.2 ml/min. The sample was loaded and PA-oligosaccharide was eluted. The result is shown in Fig. 9. The reaction product exhibited primarily two peaks with a reversed phase column and the peak of faster elution was corresponding to the elution position of Man₅GlcNAc₂-PA standard (TAKARA SHUZO CO., LTD., a structure thereof is shown in formula (III)). Thus, it was considered to be an unreacted acceptor substrate.

On the other hand, the peak of slower elution was collected, and after purification, mass analysis was performed using TOF-MS. Analysis was performed using LASERMAT2000 (ThermoQuest) and 0.01% disodium phosphate containing 2.5% 2,5-dihydroxybenzoic acid and 40% acetonitrile as a matrix. As a result, a mass of the peak fraction was corresponding to the expected molecular mass (m/z = 1521 (H⁺);m/z = 1541 (Na⁺)). Because of the strict substrate specificity of GnT-I, the resultant type sugar chain was considered to be a subject hybrid type sugar chain GlcNAcMan₅GlcNAc₂ having a structure represented by formula (IV) : 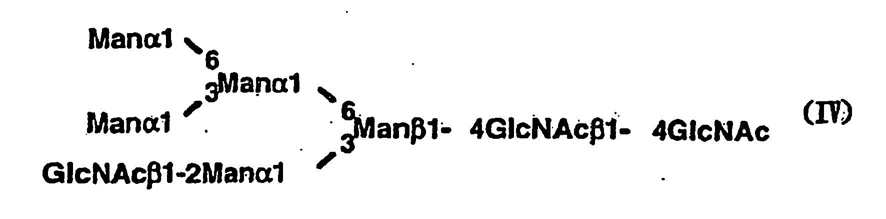 wherein Man represents mannose and GlcNAc represents N-acetylglucosamine.

### [Example 6] Introduction of α-mannosidase I gene and GnT-I gene into Δoch1Δmnn1Δmnn4 auxotrophic triple mutant

In order to biosynthesize a hybrid type sugar chain in yeast, GnT-I gene may be further introduced into a yeast strain prepared in Example 4 to express. The introduced strain can biosynthesize a mammalian hybrid type GlcNAcMan₅GlcNAc₂ sugar chain.

The ER-retrieval type expression plasmid pGAMH1 for α-1,2-mannosidase derived from *Aspergillus saitoi* (Chiba et al., J. Biol. Chem., 273, 26298-26304 (1998)) used in Example 4 was cleaved at SmaI-NaeI to cleave out a region containing a promoter, ORF of α-1,2-mannosidase subsequent to the promoter, and a terminator. This fragment was then introduced into SmaI site of pYOG4. This plasmid was designated as pYOMG4. Using this plasmid, TIY19 strain was transformed by the lithium acetate method. As a control, those transformed only by pYO354 were used. After transformation, the cells were spread on a plate containing SD-Trp medium (2% glucose, 0.67% Yeast Nitrogen Base w/o amino acids (Difco), a mixture of nucleobases and amino acids excluding tryptophan (20-400 mg/L)) and cultured at 30°C for 2 days to obtain a transformant. The resultant transformant was designated as TIY19 pYOMG4.

This auxotrophic triple mutant TIY19pYOMG4 strain in which α-mannosidase I gene and GnT-I gene have been introduced to produce a hybrid type sugar chain has been deposited internationally at the NATIONAL INSTITUTE OF BIOSCIENCE AND HUMAN-TECHNOLOGY (1-1-3, Higashi, Tsukuba, Ibaraki) as of July 2, 1999 under the accession number FERM BP-6775.

As with Example 3, a sugar chain was prepared from the resultant transformant to perform HPLC analysis.

The results of analysis by the amide column are shown in Fig. 10. A sample transformed by a control vector only exhibited primarily one peak as with the results in Example 3, which was corresponding to the elution position of ManₑGlcNAc₂-PA standard (TAKARA SHUZO CO., LTD.) (Fig. 10, A). TIY19pYOMG4 containing α-1,2-mannosidase gene and GnT-I gene exhibited primarily five peaks (Fig. 10, B). Of these five peaks, four of them (Fig. 10, B; peaks a, c, d, and e) were corresponding to the elution positions of Man₅GlcNAc₂-PA, Man₆GlcNAc₂-PA, Man₇GlcNAc₂-PA, and Man₈GlcNAc₂-PA standards. These sugar chains are referred to as human high mannose type sugar chain. Further, a new peak (Fig. 10, B; peak b), which was not found when only α-1,2-mannosidase gene was introduced, appeared. The elution position of this peak was corresponding to that of the hybrid type GlcNAcMan₅GlcNAc₂ sugar chain standard authentic sample prepared in Example 5. Further, fractions corresponding to this peak were collected and subjected to a reversed phase column as with Example 3. The column, the solvent, and the conditions were in accordance with those described in Example 5. The collected sugar chain fraction exhibited primarily one peak with a reversed phase column (Fig. 11) and this peak was again corresponding to the elution position of GlcNAcMan₅GlcNAc₂-PA standard. Therefore, it is cleared that mannoprotein produced from TIY19pYOMG4 strain contained a hybrid type GlcNAcMan₅GlcNAc₂ sugar chain.

### [Example 7] Expression of human liver α-mannosidase II in yeast

α-mannosidase II is an enzyme which converts a hybrid type sugar chain to a single-stranded complex type sugar chain in Golgi apparatus.

The nucleotide sequence of human liver α-mannosidase II gene is registered in the GenBank database under accession NO. U31520 (Misago et al., Proc. Natl. Acad. Sci., 92, 11766-11770 (1995)). Using Human Liver Marathon-Ready cDNA (Clontech) as a template, a portion coding for N-terminal region in α-mannosidase II was amplified by PCR using primer M (CGCCGCCGAGCTCTAAAAAAATGAAGTTAAGCCGCC: SEQ ID NO: 13) and primer N (ATCCCACCACTTTGAAAGGT: SEQ ID NO: 14), a portion coding for the center region was amplified by PCR using primer O (GAAGACTCACGGAGGAAGTT: SEQ ID NO: 15) and primer P (ATGGCGGTATATGTGCTCGA: SEQ ID NO: 16), and a portion coding for C-terminal region was amplified by PCR using primer Q (CGCAGTTTGGGATACAGCAA: SEQ ID NO: 17) and primer R (ATTATTATTAGCGGCCGCCCCTCAACTGGATTCG: SEQ ID NO: 18). The resultant DNA fragment was introduced into SrfI site of pCRScript. The sequence was confirmed and then recombined at BglII site to prepare an adequate sequence coding for all regions. This plasmid was designated as pCRMAN2.

In order to confirm expression of the subject protein, a gene was prepared in which 30 bp of HA tag coding for influenza virus hemagglutinin epitope was linked to 3'-terminus of α-mannosidase II gene so as to repeat three times, thereby constructing a vector. That is, a double-stranded DNA comprising sequence S (SEQ ID NO: 19) was chemically synthesized and introduced between BamHI site and EcoRI site of the expression plasmid pYEX-BX. This plasmid was designated as pYEX-BX-3HA. Subsequently, a portion coding for α-mannosidase II was cleaved out at BamHI and EcoRI of pCRMAN2 and was introduced between SacI site and NotI site of pYEX-BX-3HA. This plasmid was designated as pYEMAN2-HA.

In order to improve the expression level in yeast, a portion coding for the transmembrane region of α-mannosidase II was substituted with the transmembrane region of a gene coding for α-1,6-mannosyltransferase in yeast (OCH1). That is, a double-stranded DNA comprising sequence T (SEQ ID NO: 20) was chemically synthesized and was inserted between SacI site and EcoRI site of pBluescript. This plasmid was designated as pBOCH1. In contrast, pYMAN2-HA was adopted as a template to amplify a part of the portion coding for the catalytic region in α-mannosidase II using primer U (TTAGACTACCCATGGAACCCGCGCCGCGAGGGCTCCTTC: SEQ ID NO: 21) and primer V (CAGGAGAACTTTGGTTCGAAAAAGCTTTGACTTCTT: SEQ ID NO: 22). This sequence was confirmed and then cleaved at NcoI and HindIII to insert between NcoI site and HindIII site of pBOCH1. A fragment was cleaved out between SacI and PstI of this plasmid and substituted with the fragment between SacI and PstI of pYEMAN2-HA. This plasmid was designated as pYEOM2-HA.

*S*. *cerevisiae* wild type yeast YPH500 was used as a host and transformation was carried out by the lithium acetate method. pYEX-BX-3HA was used as a control. After transformation, the cells were spread on the plate containing SD-Ura medium (2% glucose, 0.67% Yeast Nitrogen Base w/o amino acids (Difco), a mixture of nucleobases excluding uracil and amino acids (20-400 mg/L)) and cultured at 30°C for 2 days to obtain a transformant.

The transformed yeast was cultured in SD-Ura medium at 30°C to OD660 = 0.8. An adequate amount of copper sulfate was then added thereto, and the mixture was cultured for an additional two hours. After harvesting, cells were disrupted by glass beads in SDS sample buffer. The obtained cell extract was used to perform Western blot analysis. In Western blot analysis, a rat anti-HA antibody was used as a primary antibody and an anti-rat IgG antibody-peroxidase complex was used as a second antibody, and detection was performed by exposing X-ray film adopting Super Signal Ultra as a substrate. As a result, whereas no signal at all was seen in a control, a signal was seen at a position of a molecular weight of about 140,000 in the cell extract transformed with pYEOM2-HA (Fig. 12).

Subsequently, the hybrid type sugar chain (a structure thereof is shown in formula (IV)) prepared in Example 5 was used as a substrate to measure the enzyme activity of α-mannosidase II. The hybrid type sugar chain (100 pmol, a structure thereof is shown in formula (IV)) was dried in a sample tube, and 2 µl each of 0.2 M MnCl₂, 1 M GlcNAc, and 1 M sodium acetate buffer (pH 5.6) and 8 µl of H₂O were added in the tube. Thereafter, 8 µl of cell extract was added to initiate the enzyme reaction. The reaction mixture was heated at 37°C overnight and then boiled to terminate the reaction. The mixture was centrifuged to remove an insoluble fraction and then analyzed by HPLC. HPLC analysis was carried out under the conditions used in Example 5. As a result, when the cell extract of yeast in which α-mannosidase II was expressed was used as an enzyme source, a peak corresponding to the elution point at 40 minutes clearly increased compared to the control (Fig. 13, B). The peak at 40 minutes was corresponding to the elution position of a single-stranded complex type sugar chain represented by formula (V) (Oguri et al., J. Biol. Chem., 272, 22721-22727 (1997)) : 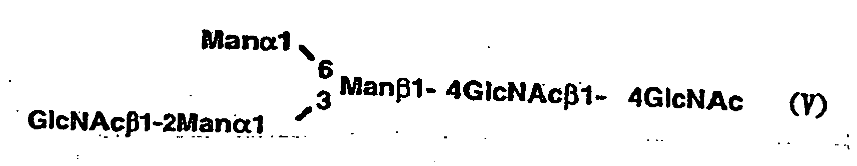 wherein Man represents mannose and GlcNAc represents N-acetylglucosamine, which was obtained from an enzyme digest of PA-sugar chain standard (PA-Sugar Chain 022, TAKARA SHUZO CO., LTD.), and thus it is confirmed that the peak corresponding to α-mannosidase II activity.

### [Example 8] Preparation of auxotrophic triple mutant into which a gene necessary for producing a double-stranded complex type sugar chain has been introduced

Expression of human GnT-II in yeast has been reported by Yoshida et al. (Yoshida S. et al., Abstracts of the meeting on Yeast Cell Biology, p. 279, Cold Spring Harbor Laboratory (1997)). GnT-II gene region containing a promoter was cleaved out at XbaI of the expression vector pSY114-GnT-II and was inserted into XbaI site of pBluescript SK. This plasmid was designated as pBlueGT2. Subsequently, GnT-I gene region containing a promoter was cleaved out at BamHI and XbaI of the plasmid pYOG4 as described in Example 6 and was inserted into BamHI site and XbaI site of pBlueGT2. The subject fragment was cleaved out at BssHII of this plasmid, bluntended by DNA T4 polymerase, and was then inserted into SmaI site of pASZ10 plasmid having ADE2 as a marker (Stotz & Linder, Gene, 95, 91-98 (1990)). This plasmid was designated as pASZGN12. pASZGN12 was linearized at HpaI and the auxotrophic triple mutant TIY19 strain prepared in Example 1 was transformed by the lithium acetate method. After transformation, the cells were spread on a plate containing SD-Ade medium (2% glucose, 0.67% Yeast Nitrogen Base w/o amino acids (Difco), a mixture of nucleobases excluding adenine and amino acids (20-400 mg/L)) containing 0.3 M KCl and cultured at 30°C for 2 days to be obtain a transformant. Genome DNA was prepared from the transformant and confirmed the incorporation of GnT-I gene and GnT-II gene into a chromosome in ADE2 region by PCR. This transformant was designated as YCY22 strain. A cell extract of YCY22 strain was used to measure respective enzyme activities, thereby confirming expression of GnT-I and GnT-II.

On the other hand, expression of human β-1,4-GalT in yeast has been reported by Yoshida et al. described above (Yoshida S. et al., Abstracts of the meeting on Yeast Cell Biology, p. 279, Cold Spring Harbor Laboratory (1997)). β-1,4-GalT gene region containing a promoter was cleaved out at SalI and XhoI of the expression vector pGalT13C and was inserted into SalI site and XhoI site of pRS403. This plasmid was designated as pRSGAL1. Expression of human UDP-Gal transporter (Ugt2p) in yeast has been reported by Kainuma et al. (Kainuma et al., Glycobiology, 9, 133-141 (1999)). A gene region containing a promoter was cleaved out at BamHI of plasmid YEp352-GAP-UGT2 for expressing the gene (UGT2) and was inserted into BamHI site of pRSGAL1. This plasmid was designated as pRSGATP1. pRSGATP1 was linearized at NdeI and YCY22 strain was transformed by the lithium acetate method. After transformation, the cells were spread on a plate containing SD-His medium (2% glucose, 0.67% Yeast Nitrogen Base w/o amino acids (Difco), a mixture of nucleobases and amino acids excluding histidine (20-400 mg/L)) containing 0.3 M KCl and cultured at 30°C for 2 days to obtain a transformant. Genome DNA was prepared from the transformant and confirmed the incorporation of β-1,4-GalT gene and UGT2 gene into a chromosome in HIS3 region by PCR. This transformant was designated as YCY42 strain. A cell extract of YCY42 strain was used to measure respective enzyme activities, thereby confirming expression of β-1,4-GalT and Ugt2p.

Subsequently, a gene fragment containing HA-tag was cleaved out at SacI and SphI from vector pYEOM2-HA for expressing human liver α-mannosidase II and bluntended by DNA T4 polymerase. This fragment was inserted into SmaI site of pAUR123. After this fragment was confirmed to be linked to a promoter in the right direction, α-mannosidase II gene region containing a promoter was cleaved out at BamHI and inserted into BamHI site of pRS406. This plasmid was linearized at NdeI and the YCY42 strain was transformed by the lithium acetate method. After transformation, the cells were spread on the plate containing SD-Ura medium (2% glucose, 0.67% Yeast Nitrogen Base w/o amino acids (Difco), a mixture of nucleobases excluding uracil and amino acids (20-400 mg/L)) containing 0.3 M KCl, and was cultured at 30°C for 2 days to obtain a transformant. Genome DNA was prepared from the transformant and confirmed the incorporation of the gene into a chromosome in URA3 region by PCR. This transformant was designated as YCY52 strain. A cell extract of YCY52 strain was used to measure the enzyme activity, thereby confirming expression of α-mannosidase II.

α-1,2-mannosidase gene fragment containing a promoter region was cleaved out at NaeI and SmaI of vector pGAMH1 for expressing α-1,2-mannosidase as described in Example 4 and was inserted into SmaI site of pY0325 vector. This plasmid was designated as pYOM5. UDP-GlcNAc transporter gene necessary for supplying a substrate to Golgi apparatus was further introduced. Expression of human UDP-GlcNAc transporter gene in yeast has been reported by Ishida et al. (Ishida et al., J. Biochem., 1261, 68-77 (1999)). This expression vector was adopted as a template to amplify an UDP-GlcNAc transporter gene region by PCR using primer W (AGAGCGGCCGCAAAATGTTCGCCAACCTAA: SEQ ID NO: 23) and primer X (TTTTGTCGACTAGACGCGTGAAGCATGCCC: SEQ ID NO: 24). This sequence was confirmed and then cleaved at NotI and SalI and substituted with the sequence between NotI site and SalI site of pG3-N. UDP-GlcNAc transporter gene fragment containing a promoter region was cleaved out at NaeI and SmaI of this plasmid and was then inserted into SmaI site of pYOM5. This plasmid was designated as pYOMR5. The YCY52 strain was transformed using this plasmid by the lithium acetate method. After transformation, the cells were spread on the plate containing SD-Leu medium (2% glucose, 0.67% Yeast Nitrogen Base w/o amino acids (Difco), a mixture of nucleobases and amino acids excluding leucine (20-400 mg/L)) containing 0.3 M KCl and cultured at 30°C for 2 days to obtain a transformant. This transformant was designated as YCY73 strain. A cell extract of YCY73 strain was used to measure the enzyme activity, thereby confirming expression of α-1,2-mannosidase and UDP-GlcNAc transporter.

A plasmid for integrating msdS and hUGTre12 was prepared. PGAMH was cleaved at Sma I and Nae I, and GAP promoter and msdS sequence were cleaved out to insert into Pvu II site of pRS404. This plasmid was designated as msdS-pRS404. A plasmid hUGTrel2-pG3, a plasmid in which hUGTrel2 was inserted downstream of GAP promoter, was cleaved at Sma I and Nae I and GAP promoter and hUGTrel2 sequence were cleaved out to insert Pst I site of msdS-pRS404. This plasmid was designated as HM-pRS404. HM-pRS404 was cleaved at BstX I in TRP1 and used for transforming YCY42 strain by the lithium acetate method. The transformant was cultured in 5 ml YPAD+0.3M KC1 at 30°C for 2 days, and incorporation of msdS and hUGTre12 into a chromosome in TRP1 was confirmed by PCR. A cell extract of the transformant was used to measure the enzyme activity, and expression of α-1,2-mannosidase and UDP-GlcNAc transporter in both strains was confirmed. The strain in which msdS and hUGTre12 were integrated into YCY42 strain was designated as TIY63 strain.

Further, a gene fragment containing HA-tag was cleaved out at SacI and SphI from vector pYEOM2-HA for expressing human liver α-mannosidase II and bluntended by DNA T4 polymerase. This fragment was inserted into SmaI site of pAUR123. After this fragment was confirmed to be linked to a promoter in the right direction, α-mannosidase II gene region containing a promoter region was cleaved out at BamHI and inserted into BamHI site of pRS406. This plasmid was linearized at NdeI and the TIY63 strain was transformed by the lithium acetate method. After transformation, the cells were spread on the plate containing SD-Ura medium (2% glucose, 0.67% Yeast Nitrogen Base w/o amino acids (Difco), a mixture of nucleobases excluding uracil and amino acids mixture (20-400 mg/L)) containing 0.3 M KCl and cultured at 30°C for 2 days to obtain a transformant. Genome DNA was prepared from the transformant and confirmed the incorporation of the gene into a chromosome in URA3 region by PCR. This transformant was designated as MSY3 strain. A cell extract of MSY3 strain was used to measure the enzyme activity, thereby confirming expression of α-mannosidase II.

### [Example 9] Preparation of auxotrophic quadruple mutant into which a gene necessary for producing a double-stranded complex type sugar chain has been introduced

At the outset, the plasmid pASZGN12 prepared in Example 8 was linearized at HpaI, and the auxotrophic quadruple mutant YS134-4A strain prepared in Example 2 was transformed by the lithium acetate method. After transformation, the cells were spread on the plate containing SD-Ade medium (2% glucose, 0.67% Yeast Nitrogen Base w/o amino acids (Difco), a mixture of nucleobases excluding adenine and amino acids (20-400 mg/L)) containing 0.3 M KCl and cultured at 30°C for 2 days to obtain a transformant. Genome DNA was prepared from the transformant and confirmed the incorporation of GnT-I gene and GnT-II gene into a chromosome in ADE2 region by PCR. This transformant was designated as YCY122 strain. A cell extract of YCY122 strain was used to measure respective enzyme activities, thereby confirming expression of GnT-I and GnT-II.

Subsequently, the plasmid pRSGATP1 prepared in Example 8 was linearized at NdeI, and YCY122 strain was transformed by the lithium acetate method. After transformation, the cells were spread on the plate containing SD-His medium (2% glucose, 0.67% Yeast Nitrogen Base w/o amino acids (Difco), a mixture of nucleobases and amino acids excluding histidine (20-400 mg/L)) containing 0.3 M KCl and cultured at 30°C for 2 days to obtain a transformant. Genome DNA was prepared from the transformant and confirmed the incorporation of β-1,4-GalT gene and UGT2 gene into a chromosome in HIS3 region by PCR. The strain was designated as YCY142 stain. A cell extract of YCY142 strain was used to measure respective enzyme activities, thereby confirming expression of β-1,4-GalT and Ugt2p.

Further, the plasmid pYOMR5 prepared in Example 8 was used to transform YCY142 strain by the lithium acetate method. After transformation, the cells ware spread on the plate containing SD-Leu medium (2% glucose, 0.67% Yeast Nitrogen Base w/o amino acids (Difco), a mixture of nucleobases and amino acids excluding leucine (20-400 mg/L)) containing 0.3 M KCl and cultured at 30°C for 2 days to obtain a transformant. This transformant was designated as YCY163 strain. A cell extract of YCY163 strain was used to measure the enzyme activity, thereby confirming expression of α-1,2-mannosidase and UDP-GlcNAc transporter.

This YCY163 strain was evaluated for lectin stainability in order to observe changes in sugar chain structure of mannoprotein on the cell surface of yeast. Concanavalin A is known to bind to specific sugar chains of high mannose type, hybrid type, double-stranded complex type and the like containing three mannose residues, and the affinity for high mannose type sugar chains is higher than that for hybrid type sugar chains and double-stranded complex type sugar chains. A solution of Texas-red labeled Concanavalin A was mixed with harvested yeast cells and was allowed to stand at 4°C for 2 hours while sporadically stirring. The reaction product was washed with PBS and then with PBS containing 10 mM α-methyl mannoside to observe under a fluorescence microscope. As a result, the periphery of a cell in YS134-4A strain was stained with fluorescence even after washing, however, fluorescence found on the periphery of a cell in YCY163 strain obviously decreased. This indicated that high mannose type sugar chains decreased while the complex type sugar chains were produced in YCY163 strain.

### [Example 10] Production of human fibroblast growth factor (FGF) in yeast mutant capable of producing mammalian type sugar chain and modification of sugar chain structure

FGF6-1 chimeric gene (secFGF (N35)) was donated by Atsuko Yoneda from the NATIONAL INSTITUTE OF BIOSCIENCE AND HUMAN-TECHNOLOGY (Yoneda et al., BioTechniques, 27, 576-590 (1999)). SecFGF(N35)/pBS was cleaved at Sma I and Nae I and FGF was cleaved out to insert into HindIII site of pGEM2-α36. This plasmid was designated as pFGFα23. pFGFα23 was cleaved at EcoR I and prepro α-factor and FGF region were cleaved out to insert into EcoR I site of pUC119 plasmid. This plasmid was designated as FGF-pUC119. In order to remove EAEA sequence of α-factor, primer Y (CGCCAGGGTTTTCCCAGTCACGAC: SEQ ID NO: 25) and primer Z (ATGGGCCGGCTCTTTTATCCAAAGATAC: SEQ ID NO: 26) were used to amplify by PCR. This DNA fragment was incorporated in EcoR I site of pUC18 to prepare pAF02 plasmid. pFGF01 was cleaved at Nae I and Sma I to cleave out FGF to insert into Nae I site and Sma I site of pAF02. This plasmid was designated as pAF03. PAF03 was cleaved at EcoR I and prepro α-factor and FGF region were cleaved out and was incorporated downstream of GAP promoter of YEp352GAP plasmid to prepare a plasmid pAFF2. pAFF2 was cleaved at Aat II and Hpa I and 2 µm region was cleaved out to construct plasmid pAFF3 for integrating yeast. Subsequently, pAFF3 was cleaved at ApaL I and Acc I to cleave out sequences of GAP promoter and FGF to insert into Pvu II site of plasmid pY0325 containing a LEU2 marker. The 2 µm region of the plasmid was further cleaved at Spe I and removed. This plasmid was designated as pAFF9. pAFF9 was cleaved at EcoR V to be linearized and yeast (TIY19 strain, YCY42 strain) was transformed by the lithium acetate method. After transformation, the cells were spread on the plate containing SD-Leu medium (2% glucose, 0.67% Yeast Nitrogen Base w/o amino acids (Difco), a mixture of nucleobases and amino acids excluding leucine (20-400 mg/L)) and cultured at 30°C for 2 days to obtain respective transformants.

These transformants were cultured in 5 ml of YPAD+0.3M KCl at 30°C for 3 days. 50 µl bed volume of heparin-Sepharose suspension (Pharmacia) was added to a culture supernatant fluid and the mixture was shaken at 4°C overnight to adsorb FGF to heparin-Sepharose. Thereafter, heparin-Sepharose was recovered by centrifugation and boiled in SDS sample buffer to subject the supernatant to SDS-PAGE. Western blotting was performed using an anti-FGF antibody to confirm FGF expression. Further, incorporation of FGF into a chromosome in LEU2 was confirmed by PCR. A strain in which FGF was integrated in TIY19 strain was designated as TIY48 strain and a strain in which FGF was integrated in YCY42 strain was designated as TIY49 strain.

In order to stably and efficiently express the protein, a plasmid for integrating msdS was prepared. Plasmid pGAMH in which msdS had been inserted downstream of GAP promoter was cleaved at EcoR I to prepare a plasmid from which 2 µm region was removed. This plasmid was designated as pImsdS. pImsdS was cleaved at Xba I in TRP1 , and TIY48 (Δmnn1::hisG Δmnn4::hisG Δochl::hisG FGF::LEU2) and TIY49 (Δmnn1::hisG Δmnn4::hisG Δoch1::hisG FGF::LEU2 ade2::[GnT-I & GnT-II] his3::[β-1.4-GalT &UGT2]) were transformed by the lithium acetate method. Each of the obtained transformants was cultured in 5 ml of YPAD+0.3M KCl at 30°C for 3 days. 50 µl of heparin-Sepharose (Pharmacia) was added to a culture solution and the mixture was shaken at 4°C overnight to adsorb FGF to heparin-Sepharose. Thereafter, heparin Sepharose was recovered. Western blotting was then performed using an FGF antibody to confirm msdS expression. Further, incorporation of msdS into a chromosome in TRP1 was confirmed by PCR. A strain in which msdS was integrated in TIY48 strain was designated as TIY53 strain and a strain in which msdS was integrated in TIY49 strain was designated as TIY54 strain.

A plasmid for integrating msdS and hUGTre12 was prepared. PGAMH was cleaved at Sma I and Nae I and GAP promoter and msdS sequence were cleaved out to insert into Pvu II site of pRS404. This plasmid was designated as msdS-pRS404. hUGTrel2-pG3, a plasmid in which hUGTre12 was inserted downstream of GAP promoter, was cleaved at Sma I and Nae I, and GAP promoter and hUGTre12 sequence were cleaved out to insert into Pst I site of msdS-pRS404. This plasmid was designated as HM-pRS404. HM-pRS404 was cleaved at BstX I in TRP1, and TIY48 strain and TIY49 strain were transformed by the lithium acetate method. Each of the transformants was cultured in 5 ml of YPAD+0.3M KCl at 30°C for 3 days. 50 µl bed volume of heparin-Sepharose suspension (Pharmacia) was added to a culture supernatant fluid and the mixture was shaken at 4°C overnight to adsorb FGF to heparin-Sepharose. Thereafter, heparin-Sepharose was recovered by centrifugation and boiled in SDS sample buffer to subject the supernatant to SDS-PAGE. Western blotting was performed using an anti-FGF antibody to confirm FGF expression. Further, incorporation of msdS and hUGTre12 into a chromosome in TRP1 was confirmed by PCR. A cell extract was used to measure the enzyme activity to confirm expression of α-1,2-mannosidase and UDP-GlcNAc transporter in both strains. A strain in which msdS and hUGTre12 were integrated in TIY48 strain was designated as TIY59 strain and a strain in which msdS and hUGTre12 were integrated in TIY49 strain was designated as TIY60 strain.

In order to prepare a sugar chain, TIY48 strain and TIY53 strain in which FGF had been integrated in a chromosome in LEU2 were used to purify FGF from 3 L of culture solution. After culturing in 3 L of YPAD+0.3 M KCl at 30°C for 3 days, 2 ml of heparin-Sepharose was added to the culture solution from which the cells were removed by centrifugation. The mixture was shaken at 4°C overnight to adsorb FGF to heparin-Sepharose. Heparin-Sepharose was recovered and packed in a column. FGF was eluted from heparin-Sepharose by increasing a salt concentration using PBS+0.01% CHAPS and PBS+2.5 M NaCl+0.01% CHAPS as solvents.

About 150 µg of purified FGF was subjected to a reversed phase column to desalt. µRPC C2/C18 PC3.2/3 column (Pharmacia) was used as a column, and 0.1% trifluoroacetic acid and 0.1% trifluoroacetic acid-60% acetonitrile were used as solvents to elute from a reversed phase column.

The sample eluted from the column was dried and hydrazinolysis was performed. 2 ml of hydrazine was added in a vacuum state, and the mixture was treated at 110°C for 60 minutes. Thereafter, the mixture was cooled to room temperature to perform N-acetylation. 250 µl of 0.2 M ammonium acetate and 25 µl of acetic anhydride were added and the mixture was thoroughly stirred and allowed to stand at room temperature for 30 minutes. Further, 250 µl of 0.2 M ammonium acetate and 25 µl of acetic anhydride were added and the mixture was thoroughly stirred and allowed to stand at room temperature for 30 minutes. The reaction solution was concentrated to dryness to prepare a sugar chain preparation.

The resultant sugar chain was subjected to the following operation for fluorescent labeling (pyridylamination). The sugar chain preparation was concentrated to dryness, and 20 µl of coupling reagent (a solution of 300 mg 2-aminopyridine in 100 µl of acetic acid) was then added thereto. The mixture was hermetically sealed and then treated at 90°C for 60 minutes. Thereafter, 20 µl of reducing reagent (a solution of 10 mg of borane-dimethylamine complex in 50 µl of acetic acid) was added. The mixture was then hermetically sealed and treated at 80°C for 60 minutes. After the reaction, 20 µl of triethylamine-methanol was added and thoroughly stirred. Thereafter, 40 µl of toluene was added thereto and thoroughly stirred. The mixture was concentrated to dryness at 60°C for 10 minutes under a nitrogen gas stream. Thereafter, 20 µl of methanol was added to the reaction solution and thoroughly stirred. 40 µl of toluene was then added thereto and thoroughly stirred. The mixture was concentrated to dryness at 60°C for 10 minutes under a nitrogen gas stream. This procedure was repeated three times. 50 µl of toluene was added to the residue to concentrate to dryness at 60°C for 10 minutes under a nitrogen gas stream. After the reaction, HW-40 gel filtration column treatment was performed to remove unreacted 2-aminopyridine.

A sugar chain structure was analyzed by HPLC using an amino column. Asahipak NH2P-50 (4.6 mm x 250 mm) was used as a column and a mixed solution of 200 mM acetic acid-triethylamine buffer (pH 7.3) and acetonitrile (7 : 3, solvent A) and a mixed solution of 200 mM acetic acid-triethylamine buffer (pH 7.3) and acetonitrile (2 : 8, solvent B) were prepared as solvents.

The column was equilibrated by running solvent A beforehand at a flow rate of 1.0 ml/min. Immediately after the sample injection, the ratio of solvent B was linearly increased to 100% over a period of 50 minutes, and then, solvent B was flowed for 20 minutes while retaining its ratio at 100%, thereby eluting PA-oligosaccharide. The result of analysis is shown in Fig. 14. The sample derived from TIY48 exhibited primarily one peak (Fig. 14, top), as with the result of Example 2, and this peak was corresponding to the elution position of Man₈GlcNAc₂-PA standard (TAKARA SHUZO CO., LTD.). Meanwhile, TIY53 strain containing α-1,2-mannosidase gene exhibited primarily one peak (Fig. 14, bottom). This peak was corresponding to the elution position of Man₅GlcNAc₂-PA standard. This indicated that FGF, which is a human glycoprotein expressed in TIY53 strain, had Man₅GlcNAC₂ sugar chain that is a precursor for nearly 100% of hybrid type and complex type sugar chains.

Further, a gene fragment containing HA-tag was cleaved out at SacI and SphI from a vector pYEOM2-HA for expressing human liver α-mannosidase II and bluntended by DNA T4 polymerase. This fragment was inserted into SmaI site of pAUR123. After this fragment was confirmed to be linked to a promoter in the right direction, α-mannosidase II gene region containing a promoter region was cleaved out at BamHI and inserted into BamHI site of pRS406. This plasmid was linearized at NdeI and the TIY60 strain was transformed by the lithium acetate method. After transformation, the cells were spread on the plate containing SD-Ura medium (2% glucose, 0.67% Yeast Nitrogen Base w/o amino acids (Difco), a mixture of nucleobases excluding uracil and amino acids (20-400 mg/L)) containing 0.3 M KCl and cultured at 30°C for 2 days to obtain a transformant. Genome DNA was prepared from the transformant and confirmed the incorporation of the gene into a chromosome in URA3 region by PCR. The transformant was designated as MSY1 strain. A cell extract of MSY1 strain was used to measure the enzyme activity, thereby confirming expression of α-mannosidase II.

### INDUSTRIAL APPLICABILITY

The newly-bred auxotrophic triple mutant and auxotrophic quadruple mutant of the present invention can produce a large quantity of high purity neutral sugar chains identical to the high mannose type sugar chains produced from human and other mammalian cells or glycoproteins having the neutral sugar chains. Also, introduction of genes for biosynthesis of a mammalian type sugar chain into the mutants enables efficient production of a mammalian type sugar chain of high-mannose type, hybrid-type, complex-type, etc. or a protein having the mammalian type sugar chain.

## Claims

1. A method for preparing a yeast mutant producing a glycoprotein having a sugar chain represented by formula (IV) set forth below: 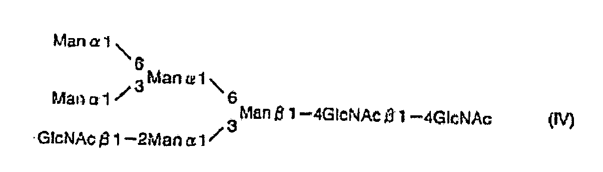 wherein Man represents mannose and GlcNAc represents N-acetylglucosamine, and wherein the method comprises the steps of:
disrupting the MNN1 gene, MNN4 gene, and OCH1 gene, in a wild-type yeast; and
introducing a gene encoding an α-mannosidase I with a C-terminal ER retention signal and a GnT-I gene, which comprises the ORF, into said yeast.

2. A method for preparing a yeast mutant, which comprises the steps of:
disrupting the MNN1 gene, MNN4 gene, and OCH1 gene, in a wild-type yeast; and
introducing a gene encoding an α-mannosidase I with a C-terminal ER retention signal, a GnT-I gene, which comprises the ORF, an α-mannosidase II gene, and a GnT-II gene into said yeast.

3. A method for preparing a yeast mutant, which comprises the steps of:
disrupting the ALG3 gene, the MNN1 gene, the MNN4 gene, and the OCH1 gene in a wild-type yeast; and
introducing a gene encoding an α-mannosidase I with a C-terminal ER retention signal into said yeast.

4. The method according to claim 3, further comprising introducing a GnT-I gene, which comprises the ORF, and a GnT-II gene into said yeast.

5. The method according to any of the preceding claims, wherein the yeast mutant has at least one auxotrophic mutation trait selected from ura3 mutation, his3 mutation, leu2 mutation, ade2 mutation, trp1 mutation, and can1 mutation.

6. The method according to any of the preceding claims, wherein the yeast mutant has a ura3 mutation.

7. The method according to any of the preceding claims, wherein the α-mannosidase I gene is derived from *Aspergillus saitoi.*

8. A method for preparing a yeast mutant according to claims 1 to 7, which comprises disrupting the OCH1 gene with a uracil marker.

9. The method according to claim 8, wherein the uracil marker is ura3.

10. A method for producing a glycoprotein having a sugar chain represented by formula (IV) set forth below: 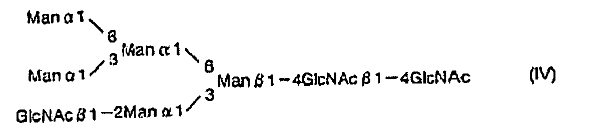 wherein Man represents mannose and GlcNAc represents N-acetylglucosamine, wherein the method comprises the steps of
- culturing the yeast mutant produced by the method according to claim 1 in a medium,
- producing and accumulating the glycoprotein in the culture product, and
- collecting the glycoprotein from the culture product.

11. A mutant yeast produced by any of the methods according to claims 1 to 9.

## Patentansprüche

1. Verfahren zur Herstellung einer Hefemutante, die ein Glycoprotein herstellt, das eine Zuckerkette, dargestellt durch die nachfolgend aufgeführte Formel (IV), aufweist: 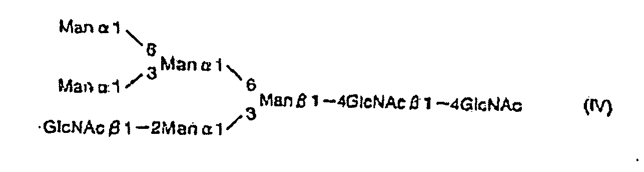 wobei Man Mannose darstellt und GlcNAc N-Acetylglucosamin darstellt, und wobei das Verfahren die Schritte umfasst:
Zerstören des MNN1-Gens, MNN4-Gens und OCH1-Gens in einer Hefe vom Wildtyp; und
Einführen eines Gens, das α-Mannosidase I mit einem C-terminalen ER-Retentionssignal codiert und eines GnT-I-Gens, das den ORF umfasst, in die Hefe.

2. Verfahren zur Herstellung einer Hefemutante, das die Schritte umfasst:
Zerstören des MNN1-Gens, MNN4-Gens und OCH1-Gens in einer Hefe vom Wildtyp; und
Einführen eines Gens, das α-Mannosidase I mit einem C-terminalen ER-Retentionssignal codiert und eines GnT-I-Gens, das den ORF umfasst, eines α-Mannosidase II-Gens und eines GnT-II-Gens, in die Hefe.

3. Verfahren zur Herstellung einer Hefemutante, das die Schritte umfasst:
Zerstören des ALG3-Gens, des MNN1-Gens, des MNN4-Gens und des OCH1-Gens in einer Hefe vom Wildtyp; und
Einführen eines Gens, das eine α-Mannosidase I mit einem C-terminalen ER-Retentionssignal codiert, in die Hefe.

4. Verfahren nach Anspruch 3, außerdem umfassend das Einführen eines GnT-I-Gens, das den ORF umfasst, und eines GnT-II-Gens in die Hefe.

5. Verfahren nach einem der vorhergehenden Ansprüche, wobei die Hefemutante mindestens eine auxotrophe Mutation aufweist, ausgewählt aus einer ura3-Mutation, his3-Mutation, leu2-Mutation, ade2-Mutation, trpl-Mutation und canl-Mutation.

6. Verfahren nach einem der vorhergehenden Ansprüche, wobei die Hefemutante eine ura3-Mutation aufweist.

7. Verfahren nach einem der vorhergehenden Ansprüche, wobei das α-Mannosidase I-Gen von Aspergillus saitoi abstammt.

8. Verfahren zum Herstellen einer Hefemutante nach den Ansprüchen 1 bis 7, das das Zerstören des OCH1-Gens mit einem Uracil-Marker umfasst.

9. Verfahren nach Anspruch 8, wobei der Uracil-Marker ura3 ist.

10. Verfahren zur Herstellung eines Glycoproteins mit einer Zuckerkette, dargestellt durch die nachfolgende Formel (IV) : 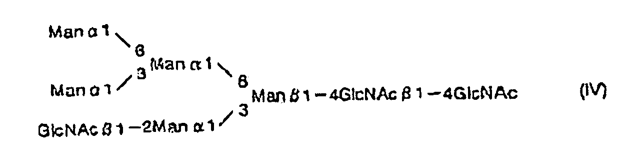 wobei Man Mannose darstellt und GlcNAc N-Acetylglucosamin darstellt, wobei das Verfahren die Schritte umfasst:
- Kultivieren der Hefemutante, hergestellt durch das Verfahren nach Anspruch 1 in einem Medium,
- Herstellen und Anhäufen des Glycoproteins in dem Kulturprodukt, und
- Sammeln des Glycoproteins aus dem Kulturprodukt.

11. Mutante Hefe, hergestellt durch eines der Verfahren nach den Ansprüchen 1 bis 9.

## Revendications

1. Procédé de préparation d'un mutant de levure produisant une glycoprotéine ayant une chaîne sucre représentée par la formule (IV) énoncée ci-dessous : 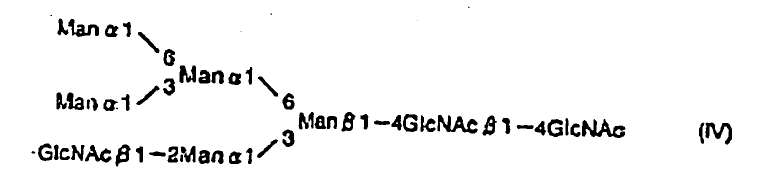 dans laquelle Man représente un mannose et GlcNAc représente une N-acétylglucosamine, et dans lequel le procédé comprend les étapes de :
disrupter le gène MNN1, le gène MNN4, et le gène OCH1, dans une levure de type sauvage ; et
introduire un gène codant pour une α-mannosidase I avec un signal de rétention ER C-terminal et un gène GnT-I, qui comprend l'ORF, dans ladite levure.

2. Procédé de préparation d'un mutant de levure, qui comprend les étapes de :
disrupter le gène MNN1, le gène MNN4, et le gène OCH1, dans une levure de type sauvage ; et
introduire un gène codant pour une α-mannosidase 1 avec un signal de rétention ER C-terminal, un gène GnT-I, qui comprend l'ORF, un gène α-mannosidase II et un gène GnT-II dans ladite levure.

3. Procédé de préparation d'un mutant de levure, qui comprend les étapes dé :
disrupter le gène ALG3, le gène MNN1, le gène MNN4, et le gène OCH1, dans une levure de type sauvage ; et
introduire un gène codant pour une α-mannosidase 1 avec un signal de rétention ER C-terminal dans ladite levure.

4. Procédé selon la revendication 3, comprenant en outre l'introduction d'un gène GnT-I, qui comprend l'ORF, et d'un gène GnT-II dans ladite levure.

5. Procédé selon l'une quelconque des revendications précédentes, dans lequel le mutant de levure a au moins un trait de mutation auxotrophe choisi parmi la mutation ura3, la mutation his3, la mutation leu2, la mutation ade2, la mutation trp1, et la mutation can1.

6. Procédé selon l'une quelconque des revendications précédentes, dans lequel le mutant de levure a une mutation ura3.

7. Procédé selon l'une quelconque des revendications précédentes, dans lequel le gène α-mannosidase I est dérivé d'*Aspergillus saitoi.*

8. Procédé de préparation d'un mutant de levure selon les revendications 1 à 7, qui comprend la disruption du gène OCH1 avec un marqueur uracile.

9. Procédé selon la revendication 8, dans lequel le marqueur uracile est ura3.

10. Procédé de production d'une glycoprotéine ayant une chaîne sucre représentée par la formule (IV) énoncée ci-dessous : 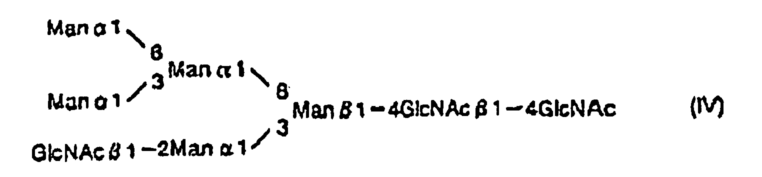 dans laquelle Man représente un mannose et GlcNAc représente une N-acétylglucosamine, et dans lequel le procédé comprend les étapes de :
- cultiver le mutant de levure produit par le procédé selon la revendication 1 dans un milieu,
- produire et accumuler la glycoprotéine dans le produit de culture, et
- collecter la glycoprotéine à partir du produit de culture.

11. Mutant de levure produit par l'un quelconque des procédés selon les revendications 1 à 9.


### SEQUENCE LISTING

<110> KIRIN BEER KABUSHIKI KAISHA
   Secretary of Agency of Industrial Science and Technology
<120> Novel yeast mutants and process for producing glycoprotein containing mammalian type sugar chain
<130> PH-1034-PCT
<160> 26
<170> PatentIn Ver. 2.0
<210> 1
   <211> 30
   <212> DNA
   <213> Artificial Sequence
<220>
   <223> Description of Artificial Sequence:synthetic DNA
<400> 1
   GGATCCGAAG AAAACCTAAT ACATTGAAGT 30
<210> 2
   <211> 36
   <212> DNA
   <213> Artificial Sequence
<220>
   <223> Description of Artificial Sequence:synthetic DNA
<400> 2
   GCATGCCCTT TGGTTTAATA TAAATCTCCG GAGTGC 36
<210> 3
   (211) 36
   <212> DNA
   <213> Artificial Sequence
<220>
   <223> Description of Artificial Sequence:synthetic DNA
<400> 3
   GCATGCTACA TAACTCCAAT CAGCAGCAAA TATGTC 36
<210> 4
   <211> 38
   <212> DNA
   <213> Artificial Sequence
<220>
   <223> Description of Artificial Sequence:synthetic DNA
<400> 4
   GCGGCCGCGT GTTCTGTTCG GGTAACGTTT AAACCAAT 38
<210> 5
   <211> 34
   <212> DNA
   <213> Artificial Sequence
<220>
   <223> Description of Artificial Sequence:synthetic DNA
<400> 5
   AGATGCATAC TAGTGGGCCC ATTGTGATTG GAAT 34
<210> 6
   <211> 30
   <212> DNA
   <213> Artificial Sequence
<220>
   <223> Description of Artificial Sequence:synthetic DNA
<400> 6
   CCCCCGAATT CGTGTGAAGG AATAGTGACG 30
<210> 7
   <211> 30
   <212> DNA
   <213> Artificial Sequence
<220> 30
   <223> Description of Artificial Sequence:synthetic DNA
<400> 7
   CCCCCGAATT CAAGTCGGAG AACCTGACTG 30
<210> 8
   <211> 34
   <212> DNA
   <213> Artificial Sequence
<220>
   <223> Description of Artificial Sequence:synthetic DNA
<400> 8
   ATGGGCCCAC TAGTATGCAT CTCGCGTGGC ATGG 34
<210> 9
   <211> 38
   <212> DNA
   <213> Artificial Sequence
<220>
   <223> Description of Artificial Sequence:synthetic DNA
<400> 9
   GCGGCCGCGA GACCTGAATC TTCGACACGC AAGAAAAA 38
<210> 10
   <211> 36
   <212> DNA
   <213> Artificial Sequence
<220>
   <223> Description of Artificial Sequence:synthetic DNA
<400> 10
   GAATTCGCTT TCGAACAAAA TCAAAAGGGG CATAAC 36
<210> 11
   <211> 32
   <212> DNA
   <213> Artificial Sequence
<220>
   <223> Description of Artificial Sequence:synthetic DNA
<400> 11
   GAATTCCTAT CCACCAAACT CACAAGCAAG CA 32
<210> 12
   <211> 38
   <212> DNA
   <213> Artificial Sequence
<220>
   <223> Description of Artificial Sequence:synthetic DNA
<400> 12
   GCGGCCGCCG AGAGGGTGAA CGGTGCTAAC TCAGGATT 38
<210> 13
   <211> 36
   <212> DNA
   <213> Artificial Sequence
<220>
   <223> Description of Artificial Sequence:synthetic DNA
<400> 13
   CGCCGCCGAG CTCTAAAAAA ATGAAGTTAA GCCGCC 36
<210> 14
   <211> 20
   <212> DNA
   <213> Artificial Sequence
<220>
   <223> Description of Artificial Sequence:synthetic DNA
<400> 14
   ATCCCACCAC TTTGAAAGGT 20
<210> 15
   <211> 20
   <212> DNA
   <213> Artificial Sequence
<220>
   <223> Description of Artificial Sequence:synthetic DNA
<400> 15
   GAAGACTCAC GGAGGAAGTT 20
<210> 16
   <211> 20
   <212> DNA
   <213> Artificial Sequence
<220>
   <223> Description of Artificial Sequence:synthetic DNA
<400> 16
   ATGGCGGTAT ATGTGCTCGA 20
<210> 17
   <211> 20
   <212> DNA
   <213> Artificial Sequence
<220>
   <223> Description of Artificial Sequence:synthetic DNA
<400> 17
   CGCAGTTTGG GATACAGCAA 20
<210> 18
   <211> 34
   <212> DNA
   <213> Artificial Sequence
<220>
   <223> Description of Artificial Sequence:synthetic DNA
<400>18
   ATTATTATTA GCGGCCGCCC CTCAACTGGA TTCG 34
<210> 19
   <211> 162
   <212> DNA
   <213> Artificial Sequence
<220>
   <223> Description of Artificial Sequence:synthetic DNA
<400> 19
   GGATCCGAGC TCCACCGCGG TGGCGGCCGC ATGTTTTACC CATACGATGT TCCTGACTAT 60
   GCGGGCTATC CCTATGACGT CCCGGACTAT GCAGGATATC CATATGACGT TCCAGATTAC 120
   GCAGCTACTA GTGGGCATGC TTCACGCGTC TAGTGAGAAT TC 162
<210> 20
   <211> 176
   <212> DNA
   <213> Artificial Sequence
<220>
   <223> Description of Artificial Sequence:synthetic DNA
<400> 20
   GAGCTCAAAA AGAAAGCAAG TAAAAGAAAG AAGAGATCAT GTCTAGGAAG TTGTCCCACC 60
   TGATCGCTAC AAGGAAATCA AAAACAATAG TCGTAACCGT ACTTCTTATT TATTCTTTGT 120
   TGACATTTCA CTTGTCAAAC AAAAGGCTGC TTTCTCAGTT TTACCCATGG GAATTC 176
<210> 21
   <211> 39
   <212> DNA
   <213> Artificial Sequence
<220>
   <223> Description of Artificial Sequence:synthetic DNA
<400> 21
   TTAGACTACC CATGGAACCC GCGCCGCGAG GGCTCCTTC 39
<210> 22
   <211> 36
   <212> DNA
   <213> Artificial Sequence
<220>
   <223> Description of Artificial Sequence:synthetic DNA
<400> 22
   CAGGAGAACT TTGGTTCGAA AAAGCTTTGA CTTCTT 36
<210> 23
   <211> 30
   <212> DNA
   <213> Artificial Sequence
<220>
   <223> Description of Artificial Sequence:synthetic DNA
<400> 23
   AGAGCGGCCG CAAAATGTTC GCCAACCTAA 30
<210> 24
   <211> 30
   <212> DNA
   <213> Artificial Sequence
<220>
   <223> Description of Artificial Sequence:synthetic DNA
<400> 24
   TTTTGTCGAC TAGACGCGTG AAGCATGCCC 30
<210> 25
   <211> 24
   <212> DNA
   <213> Artificial Sequence
<220>
   <223> Description of Artificial Sequence:synthetic DNA
<400> 25
   CGCCAGGGTT TTCCCAGTCA CGAC 24
<210> 26
   <211> 28
   <212> DNA
   <213> Artificial Sequence
<220>
   <223> Description of Artificial Sequence:synthetic DNA
<400> 26
   ATGGGCCGGC TCTTTTATCC AAAGATAC 28